(19) Europäisches Patentamt
European Patent Office
Office européen des brevets

(11) Numéro de publication : **0 539 477 B1**

# (12) FASCICULE DE BREVET EUROPEEN

(45) Date de publication du fascicule du brevet : **08.11.95 Bulletin 95/45**

(51) Int. Cl.[6] : **G01N 33/542,** G01N 33/58, G01N 33/533

(21) Numéro de dépôt : **91913539.2**

(22) Date de dépôt : **12.07.91**

(86) Numéro de dépôt international : **PCT/FR91/00567**

(87) Numéro de publication internationale : **WO 92/01225 23.01.92 Gazette 92/03**

## (54) PROCEDE D'AMPLIFICATION DU SIGNAL D'EMISSION D'UN COMPOSE LUMINESCENT.

(30) Priorité : **13.07.90 FR 9008981**

(43) Date de publication de la demande : **05.05.93 Bulletin 93/18**

(45) Mention de la délivrance du brevet : **08.11.95 Bulletin 95/45**

(84) Etats contractants désignés : **AT BE CH DE DK ES FR GB GR IT LI LU NL SE**

(56) Documents cités :
**EP-A- 0 180 492**
**WO-A-87/07955**
**WO-A-89/05813**
**GB-A- 2 223 096**
**US-A- 4 542 104**
**US-A- 4 822 733**
**WPIL, File Supplier, Accession no. 86-194289[30], Derwent Publications Ltd, (Londres, GB), & JP, A, 61128169 (MITSUBISHI CHEM. IND. K.K.) 16 juin 1986, voir l'abrégé**
**Annals of Clinical Biochemistry, vol. 18, partie 5, septembre 1981, (Londres, GB), D.S. Smith et al.: "A review of fluoroimmunoassay and immunofluorometric assay", pages 253-274, voir l'article en entier, (cité dans la demande)**
**Clinical Chemistry, vol. 31, no. 3, mars 1985, (Winston-Salem, US), I. Hemmilä: "Fluoroimmunoassays and immunofluorometric assays", pages 359-370, voir l'article en entier, (cité dans la demande)**

(56) Documents cités :
**Biochemistry, vol. 7, no. 2, février 1968, (Washington, DC, US), R.H. Conrad et al.: "Intramolecular transfer of excitation from tryptophan to 1- dimethylaminonaphthalene-5-sulfonamide in a series of model compounds", pages 777-787, voir l'article en entier, (cité dans la demande)**

(73) Titulaire : **CIS BIO INTERNATIONAL**
**RN 306**
**F-91400 Saclay (FR)**

(72) Inventeur : **MATHIS, Gérard**
**17, impasse de la Capelle-des-Ladres**
**F-30200 Bagnols-sur-Cèze (FR)**
Inventeur : **DUMONT, Christophe**
**Ruelle Cuisy**
**F-60150 Thourotte (FR)**
Inventeur : **ASPE, Daniel**
**38, rue Marcel Pagnol**
**F-30290 Laudun (FR)**
Inventeur : **FOYENTIN, Muriel**
**La Résidence 1, rue Georges-Taulier**
**F-84000 Avignon (FR)**
Inventeur : **JOLU, Etienne, Jean-Pierre**
**2, allée du Romarin**
**F-30200 Bagnols-sur-Cèze (FR)**
Inventeur : **NUTI, Dominique**
**23, rue du Chapeau-Rou**
**F-8400 Avignon (FR)**

(74) Mandataire : **Gillard, Marie-Louise**
**Cabinet Beau de Loménie**
**158, rue de l'Université**
**F-75340 Paris Cédex 07 (FR)**

**EP 0 539 477 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention a pour objet un procédé d'amplification du signal d'émission d'un composé luminescent.

Elle a également pour objet un procédé de détection et/ou de détermination par luminescence d'un analyte dans un milieu le contenant, mettant en oeuvre ce procédé d'amplification du signal d'émission d'un composé luminescent.

L'utilisation de dosages immunologiques pour l'analyse qualitative et quantitative de composés dans des fluides biologiques est à l'heure actuelle largement répandue.

Parmi les techniques existantes, les dosages par fluorimétrie ont pris une importance croissante.

En effet, ils présentent un certain nombre d'avantages parmi lesquels la sensibilité, la rapidité de la mesure, la stabilité et l'inocuité des réactifs marqués par des composés fluorescents et le coût relativement réduit.

Il est connu que les méthodes de détection utilisant la fluorescence sont intrinsèquement très sensibles et pourraient permettre des limites de détection inférieures à celles atteintes par des dosages immunologiques utilisant des réactifs radiomarqués, en particulier par l'utilisation de sources lumineuses modulables laser (I. Wieder, Immunofluorescence and related staining techniques, 1978, Elsevier).

Cependant la sensibilité de la technique dépend de nombreux paramètres.

Il ressort de l'art antérieur que la molécule fluorescente choisie comme traceur doit posséder les propriétés suivantes :

- elle doit posséder une fonction chimique permettant un couplage avec la molécule biologique sans la dénaturer ni modifier ses propriétés immunologiques ;
- le coefficient d'absorption molaire de la molécule fluorescente doit être le plus élevé possible ;
- le rendement quantique de fluorescence doit être le plus élevé possible ;
- le déplacement de Stokes doit être le plus important possible ;
- la longueur d'onde d'émission doit être si possible supérieure à 500 nm ;
- elle doit être soluble dans l'eau ou les solutions tampon.

Ces conditions sont précisées par exemple dans l'article de E. SOINI et al, Clin. Chem. 25, 353 (1979) ou dans I. HEMMILLA, Clin. Chem. 31/3, 359 (1985).

Dans le domaine des dosages immunofluorescents et en particulier des dosages immunologiques homogènes, l'une des conditions nécessaires à l'obtention d'un dosage à haute sensibilité est l'utilisation d'un marqueur fluorescent possédant un rendement quantique élevé, stable à faible dilution dans le milieu de mesure.

Par "dosage immunologique homogène", on entend un dosage dans lequel le signal de la molécule traceur est modifié lors de la liaison entre le ligand et le récepteur ou entre 2 ligands et le récepteur (l'un ou l'autre étant la molécule que l'on cherche à doser), ce qui évite de devoir séparer les molécules marquées restant libres dans le milieu de mesure de celles qui sont liées, avant d'effectuer la mesure.

Dans le cas des dosages immunologiques homogènes par fluorescence utilisant le transfert d'énergie, la condition du rendement quantique élevé du donneur est d'autant plus importante qu'il détermine également l'efficacité de transfert et par conséquent la quantité de lumière émise par l'accepteur, que l'on mesure (Ullman et al. Clinical Lab. Techniques for the 1980's, 1980, 13-43, A.R. Liss Ed.).

Ces critères de sélection du traceur luminescent s'appliquent de la même manière pour des dosages utilisant des molécules phosphorescentes.

La sensibilité de la mesure est également fortement affectée par le "bruit de fond" constitué par l'émission des autres molécules présentes dans l'échantillon à tester, susceptibles d'être excitées en même temps que le traceur luminescent et d'émettre à la longueur d'onde de mesure.

Ce problème se pose de manière particulièrement aigüe dans le cas de dosages en milieu sérique dans lequel de nombreuses molécules (protéines, etc..) sont susceptibles d'interférer dans la mesure.

Dans le cas des dosages immunofluorescents, les méthodes de mesure de fluorescence en temps résolu permettent de remédier partiellement à cet inconvénient. Le principe de ces méthodes est d' effectuer la mesure de la fluorescence émise par une molécule traceur ayant une durée de vie d'émission relativement longue, la mesure étant retardée dans le temps au-delà de la durée de vie d'émission des autres molécules présentes.

Il est dans ce cas nécessaire d'utiliser des molécules fluorescentes traceurs à durée de vie relativement longue telles que les chélates de terre rare.

Cette technique peut en particulier être utilisée dans le cas de dosages immunologiques homogènes par fluorescence utilisant un transfert d'énergie.

Le brevet US 4 822 733 décrit un procédé homogène de détection d'un analyte dans un échantillon, mettant en oeuvre un récepteur et un analyte marqués par des molécules fluorescentes ayant des durées de vie différentes. La mesure de la fluorescence résultant du transfert d'énergie est effectuée à un temps supérieur à la durée de vie d'émission de la molécule ayant la plus courte durée de vie.

Néanmoins, la mesure de fluorescence en temps résolu ne permet pas à elle seule de résoudre un des

problèmes importants posés par les dosages immunofluorescents homogènes utilisant le transfert d'énergie qui est l'existence d'un signal résiduel du traceur portant la molécule fluorescente donneur lors de la mesure à la longueur d'onde de fluorescence de l'accepteur (Morrison, Anal Biochem 174 , 119, 1988). Ce phénomène est encore plus important lorsque l'on opère avec des forts excès de traceur comme dans le cas des techniques sandwich ou lorsque l'on opère par compétition et que l'antigène marqué n'est pas pur.

Ce problème important qui limite la sensibilité a été évoqué par Ullman et al. (Clinical Lab. Techniques for the 1980's, 1980, 13-43, A.R. Liss Ed.) qui propose l'utilisation d'un anticorps anti-traceur pour inhiber le traceur libre en solution.

Ce phénomène est encore aggravé lorsque la durée de vie du donneur est longue comparée à celle mesurée sur l'accepteur.

La demande WO 87/07955 décrit plusieurs types de méthodes de dosage immunologiques par fluorescence en milieu fortement dilué, dans lequel le marqueur fluorescent est un chelate de terre rare hydrosoluble stable en milieu aqueux très dilué, et ayant une longue durée de vie. Il est expressément indiqué que les chelates utilisables doivent posséder un rendement quantique élevé ("intense quantum yield", p. 9, 1. 26).

La demande GB 2 223 096 concerne un procédé de détection d'un analyte par transfert d'énergie dans lequel l'interaction du donneur ou de l'accepteur avec l'analyte diminue l'efficacité de transfert du donneur à l'accepteur, entraînant une augmentation du signal d'émission du donneur et une diminution de celui de l'accepteur (p. 2,1. 12-26).

Le brevet US 4 542 104 décrit des conjugués fluorescents donneurs/accepteurs, dont l'une des propriétés est de posséder un rendement quantique de fluorescence élevé, dû à celui du donneur (colonne 3, 1. 49-53).

De manière inattendue, on a maintenant trouvé qu'en mettant en oeuvre, en tant que composé luminescent donneur des molécules ayant un rendement quantique global faible, et donc étant selon l'art antérieur inadéquates pour une utilisation dans un dosage, notamment un dosage immunologique par luminescence, il était possible en particulier de résoudre le problème du signal résiduel du traceur fluorescent sans nuire à l'efficacité du transfert, et par conséquent d'augmenter la sensibilité du dosage.

En effet, on a observé qu'en utilisant des composés accepteurs à des concentrations comparables à celles indiquées dans la littérature pour une utilisation avec un composé donneur à rendement quantique élevé, il était possible d'obtenir, avec un composé donneur ayant un rendement quantique global faible, une efficacité de transfert élevée et on a constaté que l'intensité du signal mesuré sur l'accepteur était supérieure à celle du signal mesuré sur le composé donneur seul. Cette constatation permet d'effectuer une mesure précise et sensible avec un composé donneur ayant un rendement quantique global faible, ceci étant surprenant compte tenu de l'analyse de l'art antérieur.

Il s'agit donc d'une méthode d'amplification du signal d'émission d'un composé luminescent donneur. De manière avantageuse, cette amplification sera d'autant plus significative que le rendement quantique global du composé luminescent donneur sera faible.

Une des hypothèses permettant d'expliquer le mécanisme de ce phénomène est de relier l'efficacité de transfert importante observée en utilisant un composé luminescent donneur ayant un rendement quantique global faible, au seul rendement de désactivation radiative du niveau émissif du donneur et non à son rendement quantique global comme enseigné dans l'art antérieur.

Dans le cas des cryptates et des chélates de terre rare utilisés comme traceurs fluorescents, on peut représenter les mécanismes photophysiques entre l'absorption et l'émission de la lumière à l'intérieur des terres rares de la manière suivante, comme indiqué par exemple dans N. Sabbatini et al., dans Supramolecular Biochemistry, 1987, p. 187-206, Reidel Dordrecht, Ed.V. Balzani.

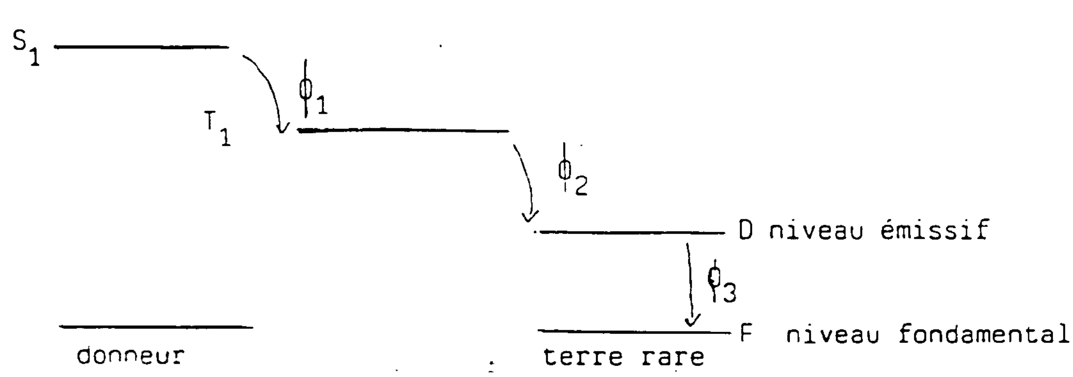


L'énergie d'excitation sert à peupler le niveau S1 qui par transition inter-système peuple le triplet. Le triplet transfère son énergie sur le niveau émissif de la terre rare qui se désactive radiativement ou non. Le rendement

global de fluorescence est le produit des rendements de ces différentes étapes :

$$\phi T = \phi 1 \times \phi 2 \times \phi 3$$

La dernière étape est la désactivation du niveau émissif de la terre rare. Elle recouvre des désactivations de différentes natures qui s'effectuent à différentes vitesses.

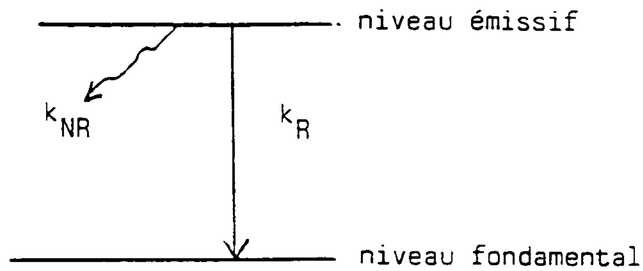


Le rendement de désactivation radiative du niveau émissif de la terre rare s'exprime par la formule

$$\phi 3 = \frac{k_R}{k_R + k_{NR}}$$

dans laquelle
$k_R$ est la vitesse de désactivation radiative et $k_{NR}$ est la somme des vitesses de désactivation non radiatives. La somme de ces 2 vitesses de désactivation s'exprime par $k_D$.

Dans le cas d'un transfert d'énergie entre un donneur et un accepteur, l'efficacité de transfert s'exprime par :

$$E = \frac{k_T}{k_T + k_R + k_{NR}} = \frac{k_T}{k_T + k_D}$$

De manière générale, il est considéré dans l'art antérieur que la vitesse de transfert $k_T$ dépend du rendement quantique global $\phi$ T. Ceci est, par exemple, indiqué dans D. Thomas et al. PNAS, 1978, 75, 5746 - 5750, par la relation

$$k_T = ko\left(\frac{r}{R_o}\right)^{-6}$$

dans laquelle
- r est la distance entre le donneur et l'accepteur,
- $R_o$ est la distance donneur-accepteur à laquelle l'efficacité de transfert est de 50 %,

et $R_o$ est fonction de $Q_o$, qui représente le rendement quantique global.

Dans le cas des molécules traceurs phosphorescentes, les mécanismes de désactivation sont analogues mais la désactivation radiative a lieu à partir du niveau triplet.

Les composés luminescents accepteurs que l'on peut utiliser pour obtenir l'amplification des signaux d'émission des composés luminescents donneurs sont choisis en fonction de ces derniers et plus précisément en fonction de leur rendement de désactivation radiative de leur niveau émissif.

En effet, on a trouvé que l'amplification du signal d'émission du composé donneur a lieu lorsque la relation

$$E\,\phi_A > \phi_3$$

dans laquelle :
- E est l'efficacité de transfert,
- $\phi_A$ est le rendement quantique du composé accepteur,
- $\phi_3$ est le rendement de désactivation radiative du niveau émissif,

est remplie.

On peut exprimer ces variables par les formules suivantes:

$$E = \frac{k_T}{k_T + k_D}$$

$$\phi_3 = \frac{k_R}{k_D}$$

$$k_T = k_R\, J\, R^{-6}\, K$$

dans lesquelles :
- E, $k_T$, $k_R$ et $k_D$ ont les significations données plus haut,
- J représente l'intégrale de recouvrement des spectres,
- R représente la distance donneur-accepteur dans l'essai considéré,

et k = $n^{-4} \times 5{,}87 \times 10^{23}$ , n étant l'indice de réfraction du milieu, lorsque R est exprimé en Å et J en $cm^3\,M^{-1}$.

E peut donc être exprimé par la relation

$$E = \frac{1}{1 + \frac{1}{\phi_3 R^{-6} KJ}}$$

la relation E $\phi_A > \phi_3$ peut être exprimée par

$$\frac{E}{\phi_3} > \frac{1}{\phi_A}$$

que l'on peut également exprimer par

$$\frac{1}{\phi_3 + \frac{1}{R^{-6}KJ}} > \frac{1}{\phi_A} \text{ ou encore } \phi_A - \phi_3 > \frac{R^6}{KJ}$$

La relation $\phi_A - \phi_3 > \frac{R^6}{5.87 \cdot 10^{23} \times n^{-4} \times J}$ régit donc le choix du composé accepteur en fonction du composé donneur car les valeurs $\phi_A$, $\phi_3$ et J sont caractéristiques du couple accepteur/donneur.

L'intégrale de recouvrement des spectres J est calculée comme indiqué dans Conrad et al, Biochemistry, 1968, 7, 777-787.

Le rendement de désactivation radiative du niveau émissif $\phi_3$ est calculé de manière connue dans la littérature, comme décrit par exemple dans N.Sabbatini et al, dans Supramolecular Biochemistry, 1987, p.187-206, Reidel Dordrecht, Ed. V. Balzani.

L'invention a donc pour objet un procédé d'amplification du signal d'émission d'un composé luminescent utilisé comme composé donneur dans un dosage par luminescence d'un analyte, dans lequel on met en oeuvre également un composé luminescent accepteur et dans lequel le signal d'émission du composé accepteur est mesuré après excitation du composé donneur à sa longueur d'onde d'excitation, caractérisé en ce que le composé donneur possède un rendement quantique global faible et en ce que le rendement quantique de désactivation radiative du niveau émissif du donneur est inférieur au rendement quantique de l'accepteur.

En tant que composé luminescent donneur mis en oeuvre dans un dosage par luminescence, on utilisera avantageusement des composés à longue durée de vie tels que des chélates ou des cryptates de terre rare fluorescents ou encore des molécules phosphorescentes.

Dans un aspect préféré, l'invention a pour objet un procédé d'amplification du signal d'émission d'un cryptate ou d'un chelate de terre rare utilisé comme composé donneur dans un dosage par luminescence d'un analyte, dans lequel on met en oeuvre également un composé luminescent accepteur et dans lequel le signal d'émission du composé accepteur est mesuré après excitation du cryptate ou du chelate de terre rare à sa longueur d'onde d'excitation, caractérisé en ce que le cryptate ou le chelate de terre rare possède un rendement quantique global faible et en ce que le rendement quantique de désactivation radiative du niveau émissif de la terre rare est inférieur au rendement quantique de l'accepteur.

Un autre aspect de l'invention concerne un procédé homogène de détection et/ ou de détermination par luminescence d'un analyte dans un milieu susceptible de le contenir, par mise en évidence du produit de la réaction entre l'analyte et au moins un récepteur correspondant, consistant :

1) à ajouter audit milieu un premier réactif constitué d'au moins un récepteur dudit analyte,

2) à ajouter un second réactif choisi parmi l'analyte ou au moins l'un de ses récepteurs, l'un des deux réactifs étant couplé avec un composé luminescent donneur constitué par un cryptate ou un chelate de terre rare et l'autre réactif étant couplé avec un composé luminescent accepteur, l'ordre d'ajout des réactifs pouvant être inversé, et, après excitation du mélange par une source de lumière à la longueur d'onde d'excitation du composé luminescent donneur,

3) à mesurer le signal d'émission du composé luminescent accepteur,

dans lequel le cryptate ou le chelate de terre rare utilisé comme composé donneur possède un rendement quantique global faible et un rendement de désactivation du niveau émissif de la terre rare inférieur au rendement quantique de l'accepteur.

Dans la présente description on définit par :

- "analyte" toute substance ou groupe de substances analogues à détecter et/ou déterminer ;
- "récepteur" toute substance capable de se fixer spécifiquement sur un site dudit analyte ;
- "composé luminescent" toute substance qui, excitée à une longueur d'onde donnée, est capable d'émettre de la lumière.

Dans un aspect préféré, le procédé homogène de détection et/ou de détermination d'un analyte dans un milieu susceptible de le contenir selon l'invention est une méthode par excès consistant;

1) à ajouter, audit milieu contenant l'analyte recherché, un premier réactif constitué par au moins un récepteur dudit analyte, couplé avec un composé luminescent donneur constitué par un cryptate ou un chelate de terre rare,

2) à ajouter un second réactif constitué par un ou plusieurs autres récepteurs dudit analyte, ledit second réactif étant couplé avec un composé luminescent accepteur,

3) à faire incuber ledit milieu après chaque addition de réactifs ou après l'addition des deux réactifs,

4) à exciter le milieu résultant à la longueur d'onde d'excitation du composé luminescent donneur,

5) à mesurer le signal émis par le composé luminescent accepteur.

Dans un aspect préféré, on utilisera dans la méthode par excès ci-dessus un seul récepteur de l'analyte qui est couplé soit avec le composé luminescent donneur, soit avec le composé luminescent accepteur.

Dans un autre aspect, ce procédé est une méthode par compétition consistant :

1) à ajouter, audit milieu contenant l'analyte recherché, un premier réactif constitué par un récepteur dudit analyte, couplé avec un composé luminescent donneur constitué par un cryptate ou un chelate de terre rare,

2) à ajouter un second réactif constitué de l'analyte couplé avec un composé luminescent accepteur,

3) à faire incuber ledit milieu après chaque addition de réactifs ou après l'addition des deux réactifs,

4) à exciter le milieu résultant à la longueur d'onde d'excitation du composé luminescent donneur,

5) à mesurer le signal émis par le composé luminescent accepteur .

Le procédé homogène de détection et/ou de détermination d'un analyte selon l'invention peut également s'appliquer à une méthode par compétition consistant :

1) à ajouter, audit milieu contenant l'analyte recherché, un premier réactif constitué par un récepteur dudit analyte, ledit récepteur étant couplé avec un composé luminescent accepteur,

2) à ajouter, un second réactif constitué de l'analyte couplé avec un composé luminescent donneur constitué par un cryptate ou un chelate de terre rare,

3) à faire incuber ledit milieu soit après l'addition de chaque réactif, soit après l'addition des deux réactifs,

4) à exciter le milieu résultant à la longueur d'onde d'excitation du composé luminescent donneur,

5) à mesurer le signal émis par le composé luminescent accepteur .

Dans un aspect avantageux, le premier réactif et le second réactif utilisés dans les procédés de détection et/ou de détermination d'un analyte indiqués ci-dessus sont ajoutés simultanément au milieu contenant l'analyte recherché.

En tant que composé luminescent donneur, on utilisera avantageusement des chélates ou des cryptates de terbium, europium, dysprosium, samarium ou neodymium. On utilisera de préférence un chélate ou un cryptate de terbium ou d'europium.

Dans un aspect avantageux, les cryptates de terre rare pouvant être mis en oeuvre dans les procédés selon l'invention sont décrits dans la demande EP 180 492. Ces composés sont constitués d'au moins un sel de terre rare complexé par un composé macropolycyclique de formule générale

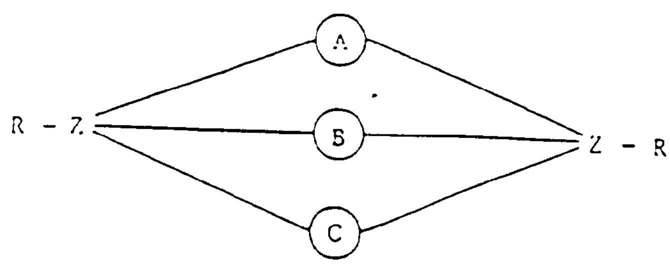

dans laquelle Z est un atome ayant trois ou quatre valences, tel que l'azote, le carbone ou le phosphore, R est rien ou représente l'hydrogène, le groupe hydroxy, un groupe amino ou un radical hydrocarboné, les radicaux bivalents Ⓐ, Ⓑ et Ⓒ sont indépendamment l'un de l'autre des chaînes hydrocarbonées qui contiennent éventuellement un ou plusieurs hétéroatomes et sont éventuellement interrompues par un hétéromacrocycle, au moins l'un des radicaux Ⓐ, Ⓑ ou Ⓒ comportant de plus au moins un motif moléculaire ou étant essentiellement constitué par un motif moléculaire, ledit motif moléculaire possédant une énergie de triplet supérieure à celle du niveau émissif de l'ion de terre rare complexé.

Des motifs moléculaires particulièrement préférés aux fins de l'invention sont la phénanthroline, l'anthracène, le benzène, le naphtalène, les bi- et ter-phényle, les bipyridines, les biquinoléines, notamment les bi-isoquinoléines, par exemple la 2,2'-bipyridine, l'azobenzène, l'azopyridine, la pyridine ou la 2,2'-bi-isoquinoléine.

A titre d'exemples de radicaux Ⓐ, Ⓑ et Ⓒ comportant un motif donneur d'énergie, on peut citer notamment les chaînes ci-après :

- $C_2H_4$ - $X_1$ - $C_6H_4$ - $X_2$ - $C_2H_4$ -

$- C_2H_4 - X_1 - CH_2 - C_6H_4 - CH_2 - X_2 - C_2H_4 -$

$X_1$ et $X_2$ pouvant être identiques ou différents désignent l'oxygène, l'azote ou le soufre ;

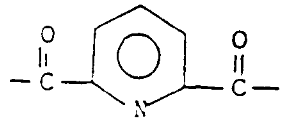

;

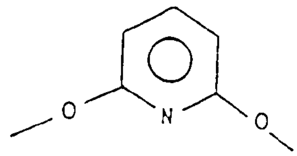

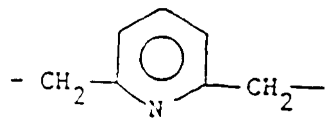

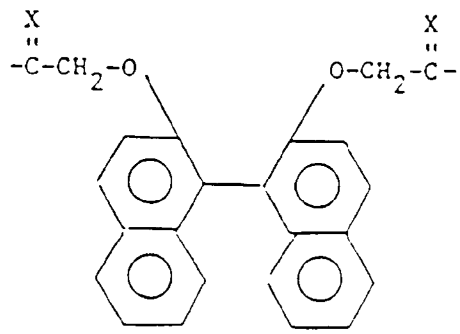

X étant l'oxygène ou l'hydrogène.

Des composés décrits dans la demande EP 0 180 492 utilisés de préférence dans le procédé de l'invention sont le cryptate de terbium Tb trisbipyridine et le cryptate d'europium Eu trisbipyridine.

D'autres cryptates de terre rare utilisables dans le procédé de l 'invention sont décrits dans la demande EP 321 353. Ils sont constitués d'au moins un sel de terre rare complexé par un composé macropolycyclique répondant à l'une des formules I ou II ci-après :

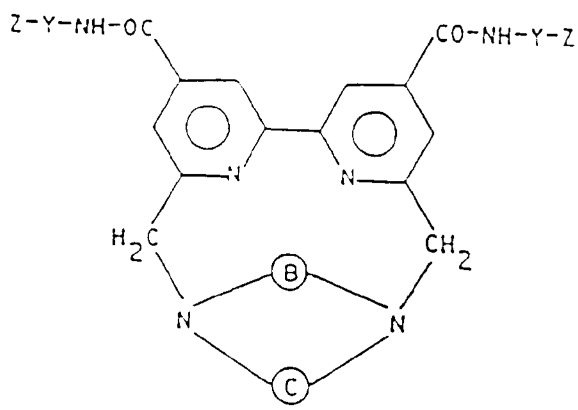

I

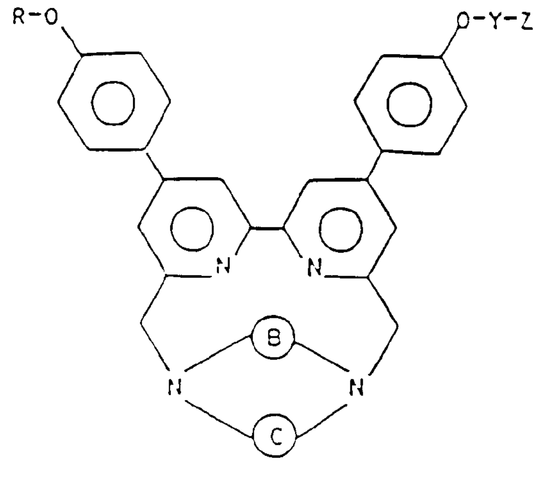

II

dans lesquels :

- le cycle de formule

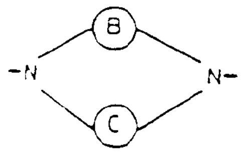

est l'un des cycles suivants :

1) 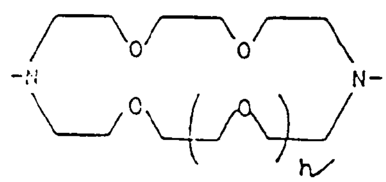

n = 0 ou 1

macrocycle $[N_2O_4]$ ou cycle (22)

macrocycle $[N_2O_3]$ ou cycle (21)

2) 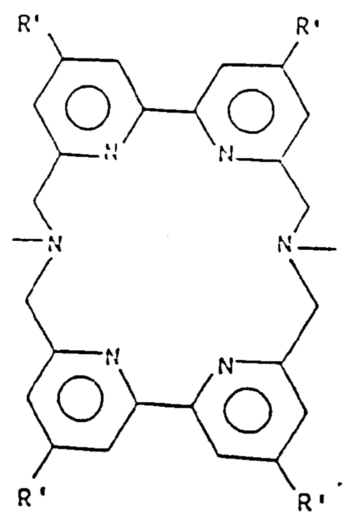

macrocycle bis-bipyridine

- Y est un groupe ou un bras d'espacement qui est constitué par un radical organique bivalent, choisi parmi les groupes alkylène linéaires ou ramifiés en $C_1$ à $C_{20}$, contenant éventuellement une ou plusieurs doubles liaisons et/ou étant éventuellement interrompus par un ou plusieurs hétéroatomes, tels que l'oxygène, l'azote, le soufre ou le phosphore, parmi les groupes cycloalkylène en $C_5$-$C_8$ ou parmi les groupes arylène en $C_6$ à $C_{14}$, lesdits groupes alkylène, cycloalkylène ou arylène étant éventuellement substitués

par des groupes alkyle, aryle ou sulfonate.

- Z est un groupe fonctionnel susceptible de se lier de façon covalente avec une substance biologique ;
- R est un groupe méthyle ou représente le groupe -Y-Z ;
- R′ est l'hydrogène ou un groupe -COOR″ dans lequel R″ est un groupe alkyle en $C_1$ à $C_{10}$ et représente de préférence le groupe méthyle, éthyle ou tertiobutyle ou bien R′ est un groupe -CO-NH-Y-Z.

A titre d'exemples de groupes fonctionnels appropriés, on peut citer notamment les groupes amino, thio, cyano, isocyano, isothiocyano, thiocyano, carboxyle, hydroxyle, maléimido, succinimido, mercapto, phénol, imidazole, aldéhyde, époxyde, halogénure, thionyle, sulfonyle, nitrobenzoyle, carbonyle, triazo, anhydride, halogénoacétate, hydrazino, acridine etc.

Les groupes particulièrement préférés sont les groupes amino, thio et carboxy qui doivent être activés avant le couplage covalent avec la substance biologique ainsi que les groupes maléimido, succinimido et isothiocyanate, lesquels peuvent se lier directement avec la substance biologique.

Des composés décrits dans la demande EP 321 353 avantageusement utilisés dans les procédés de l'invention sont le cryptate d'europium Eu trisbipyridine diamine et le cryptate de terbium Tb trisbipyridine diamine.

Les cryptates de terre rare décrits ci-dessus présentent de plus l'avantage d'avoir une longue durée de vie de l'ordre de plusieurs dizaines de $\mu s$, ce qui permet d'une part d'utiliser la technique de la mesure en temps résolu et ainsi de neutraliser les émissions parasites et, d'autre part, d'utiliser pour la mesure un matériel classique.

On peut également utiliser comme composés luminescents donneurs des composés phosphorescents tels que l'éosine ou l'érythrosine.

Les composés luminescents accepteurs utilisés dans les procédés de l'invention sont choisis en fonction des composés donneurs comme indiqué plus haut.

Lorsque le composé luminescent donneur est un cryptate d'europium fluorescent, on utilisera avantageusement un composé fluorescent accepteur choisi parmi l'allophycocyanine, l'allophycocyanine B, la C phycocyanine ou la R phycocyanine.

Dans le cas de l'utilisation d'un cryptate de terbium comme composé donneur, on utilisera avantageusement un composé fluorescent accepteur choisi parmi les rhodamines, la thionine, la R phycocyanine, la phycoerythrocyanine, la C phycoerythrine, la B phycoerythrine ou la R phycoerythrine.

On peut également utiliser comme composé luminescent donneur un composé phosphorescent tel que l'éosine ou l'erythrosine. Dans ce cas on utilisera avantageusement un composé fluorescent accepteur choisi parmi les chlorophylles telles que celles citées dans les demandes EP 71 991 et EP 314 406, ou les porphyrines telles que citées dans la demande EP 71 991 ou encore les phtalocyanines telles que celles de la demande PCT/WO 88 04777.

Dans le cas d'un dosage en milieu liquide utilisant des composés donneurs phosphorescents, la lecture sera effectuée soit sur un support solide, soit en ajoutant au milieu de mesure des molécules capteurs d'oxygène, ces techniques étant connues de l'homme du métier.

Les chlorophylles et les phtalocyanines peuvent également être utilisées comme composés accepteurs fluorescents en utilisant comme composé donneur un cryptate ou un chélate d'europium.

Comme source de lumière permettant l'excitation du composé luminescent donneur, on utilisera avantageusement une source de lumière modulable telle que celles décrites dans Lakowicz, Principles of fluorescent spectroscopy, Plenum Press, New-York, 1983, p. 96-100.

Le procédé d'amplification de l'invention trouve en particulier une application importante dans les dosages immunologiques par fluorescence, aussi bien dans les méthodes de dosage dites par compétition que par excès, en phase homogène ou hétérogène, décrits dans l'art antérieur (Landon, Ann. Clin. Biochem, 1981, 18, 253 et E. SOINI et al, Clin. Chem. 1979, 25, 353).

En particulier, le procédé d'amplification de l'invention peut être avantageusement utilisé dans les dosages immunologiques en milieu sérique nécessitant une haute sensibilité, celle-ci étant habituellement affectée par un important bruit de fond.

En effet, le procédé d'amplification de l'invention permet d'utiliser des traceurs ayant un rendement quantique global faible et dont le signal résiduel est donc également plus faible.

L'invention sera mieux comprise à l'aide des exemples ci-après qui ne présentent aucun caractère limitatif.

EXEMPLE 1 :

Deux séries d'essais d'amplification dynamique ont été réalisées en utilisant en tant que composé donneur un cryptate d'Europium Eu trisbipyridine (Eu TBP) préparé comme décrit dans la demande EP 180 492 (exemple 5) et comme composé accepteur l'allophycocyanine(Interchim, France) et d'autre part en utilisant comme composé donneur un cryptate de terbium Tb trisbipyridine (Tb TBP) préparé de manière analogue à Eu trisbipyridine et comme composé accepteur la rhodamine B (Fluka, Suisse) ou la B phycoérythrine (Sigma, USA).

Le composé donneur est utilisé à une concentration de $10^{-8}$ M/l dans différents tampons :

- tampon phosphate 0,1 M pH 7,4
- tampon Tris 0,1 M pH 8
- tampon Tris HCl 0,1 M pH 7,1

ces tampons contenant également de la serum albumine humaine à 1 g/l, en présence de différentes concentrations de composé accepteur. La fluorescence peut être mesurée en temps résolu au moyen d'un fluorimètre ARCUS (LKB, Suède) en utilisant un filtre interférentiel adapté à l'émission du composé accepteur.

Pour effectuer la mesure de la fluorescence en temps résolu dans le présent essai, on a utilisé un fluorimètre à laser prototype, qui est décrit ci-après :

Un laser pulsé à azote (LASER SCIENCE INC., modèle LS1-337ND) est utilisé comme source d'excitation (longueur d'onde à 337,1 nm). La durée des pulsations est spécifiée à 3 nanosecondes et est répétée sous une fréquence de 10 Hertz. Le faisceau passe à travers un filtre (CORNING) afin d'éliminer toute lumière parasite à l'excitation autre que 337 nm.

Après être rentré dans la chambre de mesure, le faisceau est réfléchi par un filtre dichroïque, placé à 45 degrés, qui a la propriété de réfléchir les ultraviolets et de pouvoir transmettre la lumière visible.

Le faisceau réfléchi par le filtre dichroïque est focalisé sur le puits à mesurer d'une microplaque par une lentille en silice fondue. L'émission de fluorescence est collectée selon un angle solide de 20 degrés,collimatée par la même lentille, et passe directement à travers le filtre dichroïque (fluorescence en lumière visible).

Un filtre interférentiel, de caractéristiques définies selon la longueur d'onde de fluorescence à détecter, permet de se débarrasser des lumières pouvant parasiter le signal, dont l'intensité est ensuite mesurée par un photomultiplicateur (HAMAMATSU R2949).

Le compteur de photons utilisé est un SR-400 (STANFORD RESEARCH SYSTEMS), dont les opérations et la synchronisation avec le laser sont contrôlées par un ordinateur de type IBM PC-AT via une sortie RS 232 . Les pulsations provenant du photomultiplicateur sont enregistrées pendant une fenêtre de temps ($t_g$) et après un délai ($t_d$) déterminés à condition qu'elles soient supérieures à un niveau discriminant sélectionné par le compteur de photons afin d'optimiser le rapport signal/bruit du photomultiplicateur.

Une table X-Y, pilotée par l'IBM PC-AT, permet les différents positionnements de la microplaque de mesure par des moteurs pas à pas, incluant les manoeuvres de chargement, de positionnement sous le faisceau excitant, de lecture automatique en séquentiel des 96 puits, et de sortie.

A 200 µl d'une solution de Eu TBP ou Tb TBP à 2 $10^{-8}$ M dans le tampon de travail on ajoute soit 200 µl de tampon de travail soit 200 µl des solutions suivantes :

Allophycocyanine à 1,24 $10^{-5}$ M dans du tampon de travail

Rhodamine B à 2 $10^{-5}$ M, 1,6 $10^{-5}$ M, 1,2 $10^{-5}$ M dans du tampon Tris HCl 0,1 M pH 7,1

B phycoerythrine à 1,54 $10^{-6}$ M et 9,6 $10^{-7}$ M dans du tampon Tris HCl 0,1 M pH 7,1.

Afin de déterminer l'amplification, la fluorescence a été mesurée dans un premier temps pour le composé donneur seul par mesure de la surface du pic émis à 620 nm, avec un filtre interférentiel de 80 % de transmission et de 20 nm de largeur à mi-hauteur pour Eu TBP et à 545 nm avec un filtre de 80 % de transmission et de 20 nm de largeur à mi-hauteur pour Tb TBP.

Dans un second temps, la fluorescence du mélange est déterminée après excitation à 307 nm et mesurée de la même manière mais à 670 nm pour le composé donneur en présence du composé accepteur (allophycocyanine) et à 580 nm (rhodamine, B phycoerythrine).

La lecture est effectuée à des délais de 0,1 et 0,05 ms. On mesure également la durée de vie des signaux émis.

Les résultats sont les suivants :

1) Eu trisbipyridine dans tampon phosphate 0,1 M pH 7,4

| Allophycocyanine | $\tau$ ms | signal td = 0,1 ms | A | signal td = 0,05 ms | A |
|---|---|---|---|---|---|
| 0 | 0,6 | 40 | 1 | 43,5 | 1 |
| 6,2 . 10-6M | 0,13 | 52,5 | 1,3 | 77 | 1,8 |

2) Eu trisbipyridine dans un tampon Tris 0,1 M pH 8

| Allophycocyanine | τ ms | signal td = 0,1 ms | A | signal td = 0,05 ms | A |
|---|---|---|---|---|---|
| 0 | 0,34 | 22,5 | 1 | 26 | 1 |
| 6,2 . 10-6M | 0,1 | 54 | 2,4 | 90 | 3,5 |

3) Tb trisbipyridine dans un tampon Tris HCl 0,1 M pH 7,1

| Rhodamine B | τ ms | signal td = 0,1 ms | A | signal td = 0,05 ms | A |
|---|---|---|---|---|---|
| 0 | 0,34 | 31 | 1 | 36 | 1 |
| 10-5 M | 0,086 | 106 | 3,4 | 190 | 5,3 |
| 8.10-6 M | 0,095 | 95 | 3 | 162 | 4,5 |
| 6.10-6 M | 0,115 | 85 | 2,7 | 132 | 3,7 |

4) Tb trisbipyridine dans un tampon Tris HCl 0,1 M pH 7,1

| B Phycoerythrine | τ ms | signal td = 0,1 ms | A | signal td : 0,05 ms | A |
|---|---|---|---|---|---|
| 0 | 0,34 | 31 | 1 | 36 | 1 |
| 7,7.10-7 M | 0,124 | 142 | 4,6 | 214 | 5,9 |
| 4,8.10-7 M | 0,174 | 152 | 4,9 | 200 | 5,5 |

τ = durée de vie du signal émis
td = délai de lecture
A = amplification.

Les résultats montrent que dans tous les cas on obtient une amplification du signal émis par le composé accepteur par rapport à celui du composé donneur seul. Cette amplification varie de 1,8 à 5,9.

EXEMPLE 2 :

Un essai d'amplification dynamique a été réalisé de la même manière que décrit dans l'exemple 1 en utilisant le cryptate d'europium Eu trisbipyridine (préparé selon l'exemple 5 de la demande EP 180 492) en tant que composé donneur à une concentration de $10^{-8}$ M/l et l'allophycocyanine (Cyanotech, USA) en tant que composé accepteur à une concentration de $6{,}2.10^{-6}$ M, dans de l'eau ou du tampon phosphate 0,1 M pH 7,4.

Dans un premier temps, on a déterminé le rendement de désactivation de la terre rare ($\phi_3$ Eu) dans les 2 tampons utilisés selon la formule

$$\phi_3 \text{ Eu} = \frac{\tau}{\tau_{77}}$$

dans laquelle

- $\tau$ est la durée de vie du cryptate d'europium Eu trisbipyridine mesurée dans les conditions de l'essai,
- $\tau_{77}$ est la durée de vie de ce cryptate dans l'eau lourde à 77° K (azote liquide).

Par ailleurs, on détermine le rendement quantique global ($\phi$ total) selon une méthode classique comme décrit par exemple dans Lakowicz, Principles of fluorescent spectroscopy, Plenum Press, New-York, 1983.

On a effectué ensuite la mesure de la fluorescence en temps résolu à l'aide du fluorimètre à laser-prototype décrit dans l'exemple 1 en mettant en présence le cryptate Eu trisbipyridine et l'allophycocyanine comme indiqué dans l'Exemple 1. L'efficacité de transfert a été calculée selon la référence D. Thomas et al., P.N.A.S., 1978, 75, 5746-5750 par la formule $E = 1 - \frac{\tau}{\tau_o}$, dans laquelle $\tau$ est la durée de vie du donneur en présence de l'accepteur et $\tau_o$ est la durée de vie du donneur seul.

Les résultats sont rapportés dans le tableau I ci-après:

TABLEAU I

| Milieu | $\phi$ total | $\phi_3$ Eu | Efficacité de transfert à $6{,}2.10^{-6}$M d'allophycocyanine |
|---|---|---|---|
| tampon Tris 0,1 M pH 8 | 2 % | 23 % | 70 % |
| tampon phosphate 0,1 M pH 7,4 | 4 % | 39 % | 80 % |

Ces résultats montrent qu'en utilisant un composé donneur ayant un rendement quantique total faible, il est néanmoins possible d'obtenir une efficacité de transfert élevée, ce qui entraîne une amplification du signal du composé donneur.

EXEMPLE 3 :

Un essai d'amplification dynamique a été réalisé dans les conditions de l'exemple 1 en utilisant comme composé donneur le cryptate de terbium Tb trisbipyridine préparé comme décrit dans la demande EP 180 492 et comme composés accepteurs la B phycoerythrine (Sigma, USA ) ou la rhodamine B ( Fluka,Suisse ) afin de déterminer la concentration en accepteur permettant d'obtenir une efficacité de transfert de 50 %.

Le chelate de terbium Tb $(DPA)_3$ décrit dans D. Thomas et al., P.N.A.S., 1978, 75, 5746-5750, qui possède un rendement quantique de 100 %, a été utilisé comme référence.

Les résultats montrent que les concentrations en accepteurs (B phycoerythrine ou rhodamine B) permettant d'obtenir une efficacité de transfert de 50 %, en utilisant comme donneur le cryptate de terbium Tb trisbipyridine dont le rendement quantique est voisin de 2 % sont du même ordre ( $6.10^{-7}$ M) que celles mesurées en utilisant le chelate de terbium Tb $(DPA)_3$ dont le rendement quantique est voisin de 100 %.

Ceci entraîne une amplification du signal du donneur Tb trisbipyridine.

EXEMPLE 4 : Dosage de la prolactine.

Un immuno essai homogène montrant l'application du principe de l'amplification au dosage de la prolactine a été réalisé en utilisant comme composé donneur le cryptate d'europium Eu trisbipyridine diamine préparé comme décrit dans la demande EP 321 353 (exemples 3 et 4) et comme composé accepteur l'allophycocyanine (Cyanotech, USA) couplés respectivement aux anticorps monoclonaux anti-prolactine $E_1$ et 3D3 (CIS bio international,France) reconnaissant 2 épitopes distincts de la prolactine.

Les abréviations utilisées ci-après sont les suivantes :

APC = allophycocyanine
DTT = dithiothreitol
EuTBP = cryptate d'europium Eu trisbipyridine diamine
HSA = serum albumine humaine
IgG = immunoglobuline G
SPDP = N-succinimidyl 3(2-pyridyldithio)propionate
Sulfo-SMCC = sulfosuccinimidyl 4(N-maléimidométhyl)cyclohexane 1-carboxylate.

1) PREPARATION DES IgG-3D3-APC

a) Activation de l'APC par le sulfo-SMCC

L'APC (3 mg) commercialement fournie sous forme précipitée dans une solution à 60 % de sulfate d'ammonium, est centrifugée. Après élimination du surnageant, le culot est repris par 250 μl de tampon phosphate 100 mM, pH 7,0, puis filtré à 0,8 μm afin d'éliminer les éventuelles particules en suspension.

Le filtrat est purifié par chromatographie d'exclusion sur colonne G25 superfine (Pharmacia, Suède) dans le même tampon. La concentration d'APC éluée dans le volume d'exclusion est déterminée à 650 nm, en considérant un $\varepsilon_{650nm}$ de 731000$M^{-1}$ $C_m^{-1}$.

L'activation de l'APC est réalisée en ajoutant une solution de sulfo-SMCC préparée extemporanément à raison de 6,9 mM dans un tampon phosphate 100 mM pH 7,0 et en laissant la réaction se produire pendant une heure, à température ambiante, sous agitation douce (rapport molaire de 15 à 75 sulfo-SMCC par APC). L'APC-maléimide est alors purifiée sur colonne G25 superfine en tampon phosphate 100 mM, EDTA 5 mM, pH 6,5 et conservée à 4°C avant couplage sur IgG 3D3.

b) Activation des IgG 3D3 par le SPDP

Simultanément, 5 mg d'IgG 3D3 à raison de 10 mg/ml dans un tampon phosphate 100 mM, pH 7,0 sont activés par l'ajout d'une solution de SPDP (Pierce, USA) à raison de 6,4 mM dans du dioxane dans un rapport molaire de 7,5 SPDP par IgG 3D3.

Après 35 min d'activation à température ambiante, l'IgG pyridine-2 thione est purifiée sur colonne G25 superfine dans un tampon phosphate 100 mM, EDTA 5mM, pH 6,5.

Les protéines sont concentrées et les groupes 2-pyridyl disulfides sont réduits par une solution de DTT (Sigma, USA) ayant une concentration finale de 19 mM pendant 15 min à température ambiante. Le DTT et la pyridine-2-thione sont éliminés par purification sur colonne G25 superfine en tampon phosphate 100 mM, EDTA 5mM, pH 6,5. La concentration en IgG-SH est déterminée à 280 nm avec un $\varepsilon_{280nm}$ de 210000 $M^{-1}cm^{-1}$.

c) Conjugaison des IgG 3D3-SH avec APC-maléimide

La fixation des groupements thiols sur les maléimides est réalisée en ajoutant 2,51 mg d'APC activées par mg d'IgG 3D3-SH. Après 18 heures d'incubation à 4°C et à l'obscurité sous agitation douce, les fonctions thiols restées libres sont bloquées par l'addition d'une solution à 100 mM de N-méthyl maléimide (Sigma, USA) ayant une concentration finale de 20 mM pendant une heure à température ambiante.

Le milieu réactionnel est purifiée par gel filtration sur colonne TSK G3000SW semi-préparative (Beckmann, USA ) en tampon phosphate 100 mM pH 7,0.

Les concentrations en APC et en IgG 3D3 du conjugué purifié, élué dans le premier pic, sont déterminées par les absorptions à 280 nm et à 650 nm, selon le calcul suivant :

$$[APC]_{Mole/l} = A_{650nm}/710000$$

$$[IgG]_{Mole/l} = (A_{280nm}-A'_{280nm})/210000$$

avec $A'_{280nm}$ étant la contribution à cette longueur d'onde de l'APC-maléimide, déterminée plus haut (paragraphe 1 - a)).

De l'albumine sérique humaine (HSA) est rajoutée à concurrence de 1 g/l au conjugué qui est ensuite réparti en aliquotes puis congelé à -20°C.

2) PREPARATION DES CONJUGUES IgG E1 - Eu TBP

La préparation de IgG E1-SH est réalisée selon le protocole décrit plus haut pour les IgG 3D3 mais en faisant varier le rapport molaire de 4 à 16 SPDP par IgG E1.

A 5 mg (5 $10^{-6}$moles) de Eu TBP est ajoutée une solution à 25 mM de sulfo-SMCC, en tampon phosphate 20 mM, diméthylformamide 10 % (v/v)pH 7,0 dans une proportion de 2,5 moles d'activateur par mole de EuTBP.

Après 45 min d'activation à température ambiante, le milieu réactionnel est filtré à 0,8 μm afin d'éliminer le précipité éventuellement formé. Les produits réactionnels indésirables (sulfo-SMCC, N-hydroxysuccinimide, acide (N-maléimidométhyl) carboxylique) sont éliminés par chromatographie échangeuse d'ions sur colonne Mono Q (Pharmacia, Suède) en tampon phosphate 20 mM diméthylformamide 10 % (v/v), pH 7,0 sous choc de NaCl. La concentration en Eu TBP maléimide est déterminée à 307 nm avec un $\varepsilon_{307nm}$ de 25000 $M^{-1}cm^{-1}$ ainsi que le rapport $A_{307nm}/A_{280nm}$.

De façon similaire à celle décrite plus haut on fait réagir les fonctions maléimides avec les fonctions thiols fixés sur l'anticorps, dans des proportions molaires variant de 10 à 30 Eu TBP maléimide par IgG E1-SH.

Après 18 heures d'incubation à 4°C et blocage des groupements thiols (éventuellement restés libres) par N-méthylmaléimide, le Eu TBP non couplé est éliminé par dialyse en tampon phosphate 100 mM pH 7,0 à 4°C jusqu'à épuisement (plus de fluorescence dans les bains de dialyse).

Les caractéristiques du conjugué sont déterminées par ses absorptions à 307 nm et à 280 nm en utilisant les valeurs suivantes en tenant compte de l'absorption propre du cryptate déterminée par le rapport $A_{307nm}/A_{280nm}$.

Eu TBP-maléimide :

$\varepsilon_{307nm}$ = 25000 $M^{-1}cm^{-1}$

$A_{307nm}/A_{280nm}$ : déterminée expérimentalement.

IgG E1-SH :

$\varepsilon_{280nm}$ = 210000 $M^{-1}cm^{-1}$

$\varepsilon_{307nm}$ = 0 $M^{-1}cm^{-1}$

3) APPLICATION AU DOSAGE DE LA PROLACTINE

On ajoute successivement dans des microplaques en polystyrène de 96 puits (Dynatech, USA) de 350 µl :

- 100 µl de solution standard de prolactine de concentration connue
- 100 µl de conjugué IgG E1-Eu TBP (donneur) à 0,5µg/ml
- 100 µl de conjugué IgG 3D3-APC (accepteur) à 3 µg/ml Les deux conjugués sont dilués dans du tampon phosphate 50 mM, HSA 1 g/l, pH 7,4.

Après incubation pendant une heure à température ambiante, on effectue la lecture à l'aide du fluorimètre à laser (prototype) décrit dans l'exemple 1 équipé d'un filtre à 650 nm de 20 nm de largeur, avec un délai de 50 µs et pendant 100 µs.

Les résultats obtenus, exprimés en coups par seconde (cps), sont rapportés dans le tableau II ci-après :

TABLEAU II

| | | cps | Δ cps |
|---|---|---|---|
| tampon HSA | | 4890 | |
| prolactine | 0 | 9579 | 0 |
| | 1200 µU | 13638 | 4059 |
| | 3000 µU | 17638 | 8059 |
| | 6500 µU | 23108 | 13529 |

Ces résultats montrent que le signal mesuré résultant du transfert donneur-accepteur varie proportionnellement à la concentration en prolactine dans le milieu.

Les composés donneurs à faible rendement quantique selon l'invention sont donc particulièrement adaptés à ce type de dosage immunologique.

EXEMPLE 5 : Dosage de la prolactine .

Un autre essai a été réalisé de manière analogue à celle décrite ci-dessus dans l'exemple 4, paragraphe 3), en utilisant un composé accepteur différent : la C phycocyanine (Cyanotech,USA) .

On ajoute successivement dans des microplaques en polystyrène de 96 puits (Dynatech, USA ) de 350 µl :

- 100 µl de solution standard de prolactine de concentration connue
- 100 µl de conjugué IgG E1-Eu TBP (donneur) à 0,1 ou 0,5 µg/ml
- 100 µl de conjugué IgG 3D3-Phycocyanine à 3 µg/ml.

Les deux conjugués sont dilués dans du tampon phosphate 50 mM, HSA 1 g/l, pH 7,4.

La mesure est effectuée après une heure d'incubation à température ambiante à l'aide du fluorimètre à laser décrit dans l'exemple 1 équipé d'un filtre à 647 nm de 20 nm de largeur.

Les résultats sont donnés dans le tableau III ci-après et exprimés en coups par seconde (cps) :

TABLEAU III

| | cps | Δ cps |
|---|---|---|
| tampon HSA | 4890 | |
| - IgG E1-Eu TBP 0,1 g/ml | | |
| Prolactine 0 | 5543 | 0 |
| 250 μU | 6525 | 982 |
| 1000 μU | 7731 | 2188 |
| 10000 μU | 8595 | 3052 |
| - IgG E1-Eu TBP 0,5 g/ml | | |
| Prolactine 0 | 9266 | 0 |
| 250 μU | 10359 | 1086 |
| 1000 μU | 15010 | 5744 |
| 10000 μU | 22805 | 13539 |

Ces résultats montrent à nouveau la variation du signal mesuré résultant du transfert donneur-accepteur, proportionnellement à la quantité de prolactine présente dans le milieu.

EXEMPLE 6 : Dosage de l'antigène 19.9.

L'antigène 19.9 est un carbohydrate représentatif du carcinome du colon.

Le site de l'anticorps anti-19.9 étant un épitope répétitif, on marque le même anticorps soit avec le donneur, soit avec l'accepteur.

On utilise pour cet immuno-essai homogène des conjugués anticorps - Eu TBP et anticorps -APC préparés de manière analogue à celle décrite dans l'exemple 4.

L'antigène 19.9 et l'anticorps anti-19.9 sont fournis par Centochor, USA.

Les deux conjugués sont dilués dans un tampon phosphate 100 mM, Na F 150 mM, HSA 1 g/l, pH 6.

On réalise une gamme standard d'antigène 19.9 par dilution d'une solution concentrée d'antigène dans du sérum de veau nouveau-né.

On ajoute successivement dans des microplaques en polystyrène de 96 puits (Dynatech, USA) :

- 50 μl de solution standard d'antigène 19.9
- 50 μl de tampon de dilution
- 100 μl de conjugué anticorps-Eu TBP à 0,5 μg/ml
- 100 μl de conjugué anticorps-APC à 5μg/ml.

Après incubation pendant 3 h30 à température ambiante, on effectue la lecture à l'aide du fluorimètre à laser décrit dans l'exemple 1 équipé d'un filtre à 650 nm de 20 nm de largeur, avec un délai de 50μs pendant 400μs.

Les résultats sont rapportés dans le tableau IV ci-après et exprimés en unités arbitraires (UA).

TABLEAU IV

| Antigène 19.9 u/ml | Signal UA |
|---|---|
| 0 | 312 |
| 9 | 419 |
| 30 | 572 |
| 64 | 890 |
| 138 | 1602 |
| 278 | 2491 |

Ces résultats montrent que le signal mesuré varie proportionnellement à la quantité d'antigène 19.9 dans le milieu.

EXEMPLE 7 : Dosage de l'antigène carcinoembryonnaire (ACE).

Dans cet immuno-essai homogène, on utilise deux anticorps monoclonaux G12 et G15 (CIS bio international, France) couplés respectivement avec du cryptate d'europium Eu TBP et de l'allophycocyanine.

Les deux conjugués G12-Eu TBP et G15-APC sont dilués dans un tampon phosphate 100 mM, HSA 1 g/l, NaF 150 mM.

On ajoute successivement dans des microplaques en polystyrène (Dynatech, USA) :

- 100 µl de solution standard
- 100 µl de conjugué G12-cryptate à 0,5µg/ml
- 100 µl de conjugué G15-APC à 5µg/ml.

Après incubation pendant 3 h à 37°C on effectue la lecture à l'aide d'un fluorimètre à laser décrit dans l'exemple équipé d'un filtre à 650 nm de 20 nm de largeur, avec un délai de 50 µs pendant 400 µs.

Les résultats obtenus sont rapportés dans le tableau V ci-après et exprimés en unités arbitraires (UA) :

TABLEAU V

| ACE ng/ml | Signal UA |
|---|---|
| 0 | 345 |
| 5,9 | 420 |
| 21 | 474 |
| 72 | 769 |
| 2634 | 1058 |

Les résultats montrent une variation du signal émis proportionnelle à la concentration en ACE. De plus, l'amplification du signal permet de détecter l'ACE avec une sensibilité de l'ordre du ng/ml.

EXEMPLE 8 :

Dosage de la digoxine

La digoxine est un glucoside cardiotonique utilisé comme principe actif de médicaments traitant l'insuffisance cardiaque.

A/ Préparation d'un traceur allophycocyanine digoxine par couplage au periodate.

a) activation de la digoxine par le periodate

268 µl d'une solution de $NaIO_4$ (Fluka, Suisse) préparée extemporanément sont ajoutés à une suspension de digoxine (ref 37100 Fluka, Suisse) de 5,35 mg dans 268 µl d'éthanol absolu.

Le mélange est incubé pendant 20 min à température ambiante sous agitation, puis la réaction est bloquée par ajout de 100 µl de glycérol 0,1 M.

La concentration finale en digoxine activée calculée sur la masse initiale et le volume final est de $1,2 \times 10^{-2}$M.

b) couplage de la digoxine activée avec l'allophycocyanine (APC)

A 2 ml de solution APC purifiée (Cyanotech, USA) en tampon borate pH 9,0 sont ajoutés 95 µl de la solution de digoxine activée au periodate.

Le mélange est incubé pendant 1h30 à température ambiante sous agitation douce. La réaction est bloquée par ajout de 100 µl d'une solution de borohydrure de sodium préparée extemporanément à raison de 5 mg de $NaBH_4$ dans 1,32 ml de tampon borate pH 9,0.

c) purification du traceur APC-digoxine

2 ml du mélange réactionnel sont injectés sur une colonne HR 10/10 G25 (Pharmacia, Suède) équilibrée en tampon phosphate 100 mM, pH 7, et élués dans le volume exclu. Les réactifs en excès sont élués par 8 ml de tampon.

On récupère environ 4 ml de solution de produit couplé APC-digoxine à 1,22 mg/ml en APC (mesurée par l'absorbance à 650 nm).

La concentration en digoxine est évaluée par un dosage RIA ou DELFIA (Pharmacia, Suède).

Le rapport molaire final calculé digoxine/allophycocyanine est d'environ 0,8.

B/ Dosage homogène compétitif de la digoxine en sérum humain.

On prépare un conjugué anticorps-cryptate d'europium Eu TBP diamine de manière analogue à celle décrite dans l'exemple 1, en utilisant un anticorps monoclonal de souris anti-digoxine (ref. G 31604 M, Interchim, France). Ce conjugué anticorps-cryptate est utilisé à la concentration de 0,5 µg/ml en tampon phosphate 100 mM, NaF 150 mM, HSA 1 g/l, pH 7.

Le traceur APC-digoxine est utilisé à une concentration de 0,2 µg/ml dans le même tampon.

On prépare une gamme de standards digoxine par dilution en sérum humain d'une solution de digoxine (Fluka, Suisse) à 1 mg/ml dans un mélange éthanol/eau 50/50.

La courbe standard estréalisée à l'aide de prises de 250 µl contenant

- 50 µl de tampon phosphate
- 50 µl de standard
- 100 µl de traceur APC-digoxine
- 100 µl de conjugué anticorps-cryptate.

Les échantillons à doser sont composés de la même façon en remplaçant les 50 µl de standard par 50 µl de sérum individuel à doser.

Le dosage est réalisé dans des microplaques de 96 puits (Dynatech, USA).

Après 30 min d'incubation à température ambiante, on effectue la lecture sur un fluorimètre à laser tel que décrit dans l'exemple 1, équipé d'un filtre à 665 nm, de 20 nm de largeur à mi-hauteur, dans une fenêtre de temps de 100 µs, après un délai de 50 µs.

Les résultats sont exprimés en pourcentage du rapport $B/B_o$ dans lequel B représente la valeur obtenue pour chaque standard et $B_o$ elle du standard 0.

Les résultats obtenus sont rapportés dans le tableau VI ci-après :

TABLEAU VI

| Standard digoxine ng/ml | $B/B_o$ % |
|---|---|
| 0 | 100 |
| 1,6 | 96,21 |
| 3,2 | 87,15 |
| 6,4 | 73,57 |
| 12,8 | 56,24 |
| 26 | 48,86 |

Ces résultats montrent que les valeurs obtenues varient proportionnellement à la concentration en digoxine.

EXEMPLE 9 :

Dosage de la thyroxine.

A/ Preparation d'un traceur allophycocyanine-thyroxine (APC-$T_4$)

a) Activation de la thyroxine par le S-AMSA

A 500 µl d'une solution à 20 mg/ml de $T_4$ (Calbiochem, France) dans du méthanol sont ajoutés 500 µl d'une solution de S-AMSA (Sigma, USA) à 8,7 mg/ml (50mM) dans ou méthanol et le mélange est incubé 15 mn à température ambiante, avant ajoût de 500 µl d'une solution d'hydroxylamine à 6,95 mg/ml (100 mM) dans du méthanol. Après 15 min d'incubation à température ambiante, 975 µl du mélange réactionnel sont prélevés et additionnés de 525 µl d'eau bidistillée filtrée sur filtre MILLIPORE HA 0,45 µm. Cette solution est passée par fractions de 250 µl sur une colonne HPLC Pep.RPC HR 5/5 (Pharmacia, Suède) et éluée par un mélange méthanol/eau 65/35. Les trois premiers pics élués sont récupérés, et les fractions successives sont jointes et évaporées sous vide dans un évaporateur rotatif. On récupère environ 3 mg de $T_4$ activée ($T_4$-SH).

b) Activation de l'allophycocyanine par le SMCC

10 mg d'APC sont purifiés, puis concentrés sur cône AMICON CENTRICON 30 jusqu'à une concentration de 10,6 mg/ml sous 700 µl.

A cette solution sont ajoutés 160 µl d'une solution de sulfo-SMCC (Pierce, USA) à 10 mg/ml dans un tampon phosphate 100 mM, pH 7. Le mélange réactionnel est incubé 1 h à température ambiante sous agitation, puis l'APC activée est purifiée par chromatographie d'exclusion sur colonne G 25 HR 10/10 (Pharmacia, Suède) en tampon phosphate pH 7.

c) Couplage de la thyroxine activée avec l'allophycocyanine activée

Le contenu du ballon de $T_4$-SH est repris par 200 µl de méthanol 50 µl de cette solution sont ajoutés à 2 ml de solution d'APC activée en tampon phosphate 100 mM, pH 7. Le mélange réactionnel est incubé 18 h à 4°C sous agitation, puis les sites maléimides en excès sont bloqués par 5 µl d'une solution de mercaptoéthanol (Sigma, USA) au 1/10 dans le tampon phosphate.

d) Purification du traceur APC-$T_4$

Le mélange réactionnel est concentré jusqu'à un volume de 1 ml sur dispositif d'ultrafiltration AMICON CENTRICON 30, puis 50 µl d'une solution de N-AANS à 1 mg/ml dans le tampon phosphate sont ajoutés (concentration finale en N-AANS environ 50 µg/ml). Le traceur est finalement purifié sur colonne de chromatographie d'exclusion PHARMACIA G 25 HR 10/10 (tampon d'élution phosphate 100 mM pH 7). On obtient 2,5 ml environ de traceur à la concentration (calculée par DO à 650 nm, $\varepsilon$ = 731 000) de 0,8 mg/ml.

L'évaluation du nombre de molécules de $T_4$ couplées par molécule d'APC est réalisée par dosage RIA du traceur et comparaison à une courbe standard en $T_4$ (trousse $T_4$-KPR, ORIS Industrie, France). Le calcul donne un rapport sur le traceur purifié de 1,4 $T_4$/APC.

La purification du traceur APC-$T_4$ s'effectue en présence de N-AANS synthétisé à partir de N-ANS

(KODAK, USA) par une modification du protocole décrit par HINDS et al. (CLIN. CHEM. 32, 16-21, 1986).

B/ Dosage homogène compétitif de la thyroxine en sérum humain

On prépare un conjugué anticorps-cryptate d'europium Eu TBP diamine de manière analogue à celle décrite dans l'exemple 1, en utilisant un anticorps monoclonal de souris anti-$T_4$ $R_{41}$ (CIS bio international, France). Ce conjugué est utilisé à la concentration de 2 µg/ml en tampon phosphate 100 mM, NaF 120 mM, HSA 0,2 %, pH 7.

On prépare une gamme de standards $T_4$ par dilution dans un sérum humain normal traité par échange d'ions et dépourvu de $T_4$. La courbe standard est réalisée à l'aide de prises de 250 µl contenant

- 50 µl de solution de N-AANS
- 50 µl de sérum humain dépourvu de $T_4$ ou 50 µl de l'un des standards $T_4$
- 100 µl de traceur APC-$T_4$
- 100 µl de conjugué anticorps-cryptate.

Les échantillons à doser sont composés de la même manière, en remplaçant les 50 µl de standard par 50 µl de sérum individuel à doser.

Le dosage est réalisé dans des microplaques de 96 puits (Dynatech, USA).

Après incubation pendant 30 mn à température ambiante, on effectue la lecture sur un fluorimètre à laser tel que décrit dans l'exemple 1 équipé d'un filtre à 665 nm, de 20 nm de largeur à mi-hauteur, en utilisant une lampe Flash 1000 Hz comme source d'excitation pendant 1 s, dans une fenêtre de temps de 100 µs, après un délai de 50 µs.

Les valeurs obtenues pour chaque standard (B) sont divisées par la valeur du standard 0 ($B_o$) et exprimées en pourcentage ($B/B_o$ %). La concentration des échantillons à doser estcalculée par comparaison avec la courbe standard.

Les résultats sont rapportés dans le tableau VII ci-après:

TABLEAU VII

| thyroxine ng/ml | $B/B_0$ % |
|---|---|
| 0 | 100 |
| 10 | 91 |
| 20 | 82,6 |
| 50 | 61,7 |
| 100 | 43,3 |
| 250 | 23 |

Les résultats montrent que les valeurs obtenues varient proportionnellement à la concentration en $T_4$.

## Revendications

1. Procédé d'amplification du signal d'émission d'un composé luminescent utilisé comme composé donneur dans un dosage par luminescence d'un analyte, dans lequel on met en oeuvre également un composé luminescent accepteur et dans lequel le signal d'émission du composé accepteur est mesuré après excitation du composé donneur à sa longueur d'onde d'excitation, caractérisé en ce que le composé donneur possède un rendement quantique global faible et en ce que le rendement quantique de désactivation radiative du niveau émissif du donneur est inférieur au rendement quantique de l'accepteur.

2. Procédé d'amplification du signal d'émission d'un cryptate ou d'un chelate de terre rare utilisé comme

composé donneur dans un dosage par luminescence d'un analyte, dans lequel on met en oeuvre également un composé luminescent accepteur et dans lequel le signal d'émission du composé accepteur est mesuré après excitation du cryptate ou du chelate de terre rare à sa longueur d'onde d'excitation, caractérisé en ce que le cryptate ou le chelate de terre rare possède un rendement quantique global faible et en ce que le rendement quantique de désactivation radiative du niveau émissif de la terre rare est inférieur au rendement de l'accepteur.

**3.** Procédé homogène de détection et/ ou de détermination par luminescence d'un analyte dans un milieu susceptible de le contenir, par mise en évidence du produit de la réaction entre l'analyte et au moins un récepteur correspondant, consistant :
1) à ajouter audit milieu un premier réactif constitué d'au moins un récepteur dudit analyte,
2) à ajouter un second réactif choisi parmi l'analyte ou au moins l'un de ses récepteurs, l'un des deux réactifs étant couplé avec un composé luminescent donneur constitué par un cryptate ou un chelate de terre rare et l'autre réactif étant couplé avec un composé luminescent accepteur, l'ordre d'ajout des réactifs pouvant être inversé et, après excitation du mélange par une source de lumière à la longueur d'onde d'excitation du composé luminescent donneur,
3) à mesurer le signal d'émission du composé luminescent accepteur,
caractérisé en ce que le cryptate ou le chelate de terre rare utilisé comme composé donneur possède un rendement quantique global faible et en ce que le rendement de désactivation radiative du niveau émissif de la terre rare est inférieur au rendement quantique de l'accepteur.

**4.** Procédé selon la revendication 3 consistant en une méthode par excès, caractérisé en ce qu'il consiste :
1) à ajouter, audit milieu contenant l'analyte recherché, un premier réactif constitué par au moins un récepteur dudit analyte, couplé avec un composé luminescent donneur constitué par un cryptate ou un chelate de terre rare,
2) à ajouter un second réactif constitué par un ou plusieurs autres récepteurs dudit analyte, ledit second réactif étant couplé avec un composé luminescent accepteur,
3) à faire incuber ledit milieu après chaque addition de réactifs ou après l'addition des deux réactifs,
4) à exciter le milieu résultant à la longueur d'onde d'excitation du composé luminescent donneur,
5) à mesurer le signal émis par le composé luminescent accepteur.

**5.** Procédé selon la revendication 3 consistant en une méthode par compétition, caractérisé en ce qu'il consiste :
1) à ajouter, audit milieu contenant l'analyte recherché, un premier réactif constitué par un récepteur dudit analyte, couplé avec un composé luminescent donneur constitué par un cryptate ou un chelate de terre rare,
2) à ajouter un second réactif constitué de l'analyte couplé avec un composé luminescent accepteur,
3) à faire incuber ledit milieu après chaque addition de réactifs ou après l'addition des deux réactifs,
4) à exciter le milieu résultant à la longueur d'onde d'excitation du composé luminescent donneur ,
5) à mesurer le signal émis par le composé luminescent accepteur .

**6.** Procédé selon la revendication 3 consistant en une méthode par compétition, caractérisé en ce qu'il consiste :
1) à ajouter, audit milieu contenant l'analyte recherché, un premier réactif constitué par un récepteur dudit analyte, ledit récepteur étant couplé avec un composé luminescent accepteur,
2) à ajouter, un second réactif constitué de l'analyte couplé avec un composé luminescent donneur constitué par un cryptate ou un chelate de terre rare,
3) à faire incuber ledit milieu soit après l'addition de chaque réactif, soit après l'addition des deux réactifs,
4) à exciter le milieu résultant à la longueur d'onde d'excitation du composé luminescent donneur,
5) à mesurer le signal émis par le composé luminescent accepteur .

**7.** Procédé selon l'une quelconque des revendications 3 à 6, caractérisé en ce que le premier réactif et le second réactif sont ajoutés simultanément au milieu contenant l'analyte recherché.

**8.** Procédé selon l'une quelconque des revendications 3 ou 4, caractérisé en ce qu'on utilise un seul récepteur de l'analyte, qui est couplé soit avec le composé luminescent donneur, soit avec le composé luminescent accepteur.

**9.** Procédé selon l'une quelconque des revendications 1 à 8, caractérisé en ce que le composé luminescent donneur est un chélate ou un cryptate de terbium ou d'europium.

**10.** Procédé selon la revendication 9 , caractérisé en ce que le composé donneur est un cryptate de terre rare constitué d'au moins un sel de terre rare complexé par un composé macropolycyclique de formule générale :

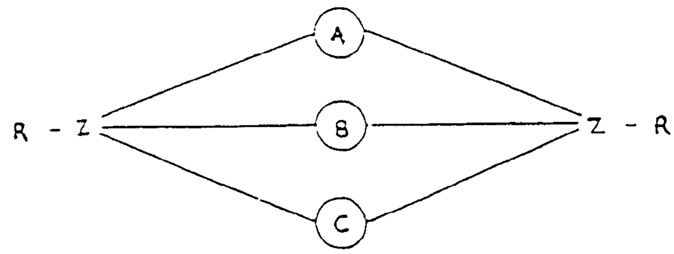

dans laquelle Z est un atome ayant trois ou quatre valences, tel que l'azote, le carbone ou le phosphore, R est rien ou représente l'hydrogène, le groupe hydroxy, un groupe amino ou un radical hydrocarboné, les radicaux bivalents Ⓐ , Ⓑ et Ⓒ sont indépendamment l'un de l'autre des chaînes hydrocarbonées qui contiennent éventuellement un ou plusieurs hétéroatomes et sont éventuellement interrompues par un hétéromacrocycle, au moins l'un des radicaux Ⓐ , Ⓑ ou Ⓒ comportant de plus au moins un motif moléculaire ou étant essentiellement constitué par un motif moléculaire, ledit motif moléculaire possédant une énergie de triplet supérieure à celle du niveau émissif de l'ion de terre rare complexé.

**11.** Procédé selon la revendication 9, caractérisé en ce que le composé cryptate de terre rare est complexé par un composé macropolycyclique de formule générale :

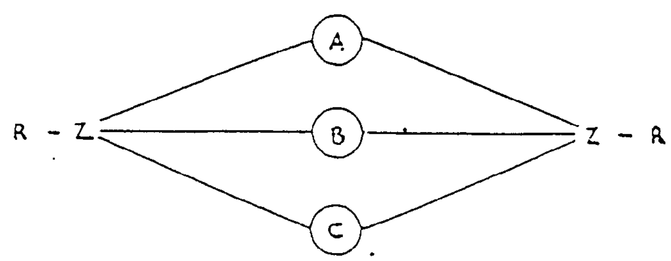

dans laquelle Ⓐ , Ⓑ et Ⓒ ont les significations suivantes :

$- C_2H_4 - X_1 - C_6H_4 - X_2 - C_2H_4 -$

$- C_2H_4 - X_1 - CH_2 - C_6H_4 - CH_2 - X_2 - C_2H_4 -$

$X_1$ et $X_2$ pouvant être identiques ou différents désignent l'oxygène, l'azote ou le soufre ;

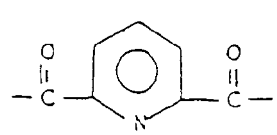 ; 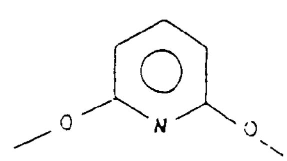

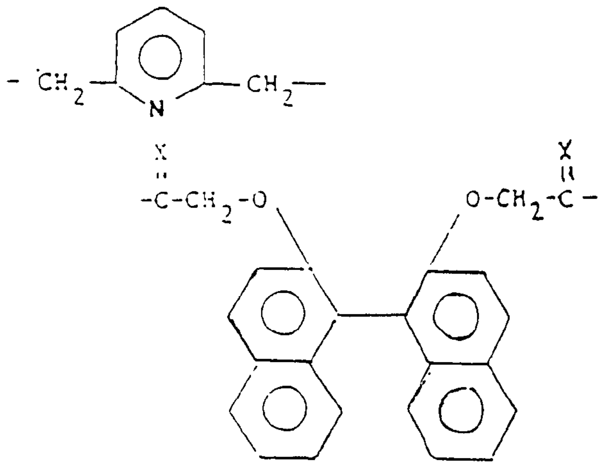

X étant l'oxygène ou l'hydrogène.

**12.** Procédé selon les revendications 9 ou 10, caractérisé en ce que le composé donneur est le cryptate de terbium Tb trisbipyridine ou le cryptate d'europium Eu trisbipyridine.

**13.** Procédé selon la revendication 8, caractérisé en ce que le composé luminescent donneur est un cryptate de terre rare constitué d'au moins un sel de terre rare complexé par un composé macropolycyclique de formule I ou II :

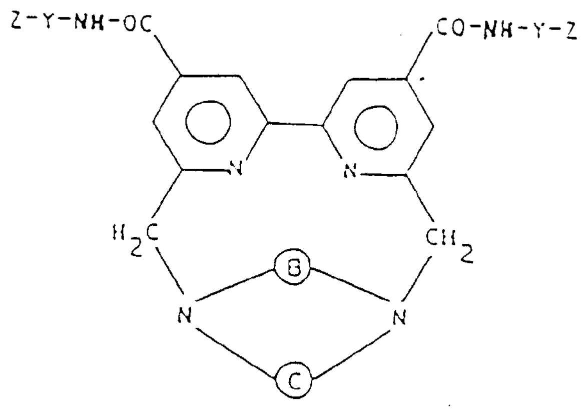

I

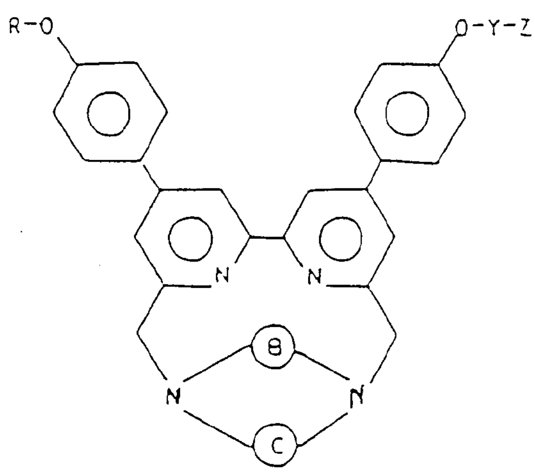

II

dans lesquels :

- Le cycle de formule

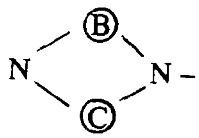

est l'un des cycles suivants :

1) 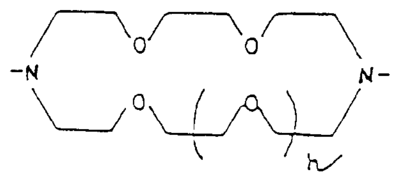

n = 0 ou 1

macrocycle $[N_2O_4]$ ou cycle (22)

macrocycle $[N_2O_3]$ ou cycle (21)

2) 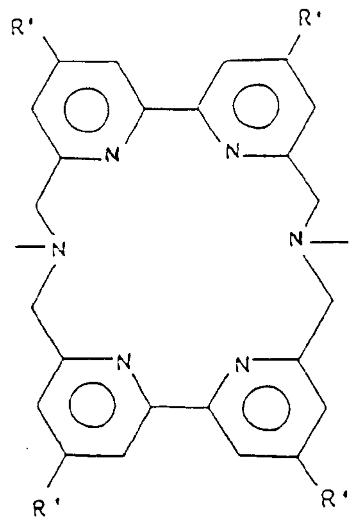

macrocycle bis-bipyridine

- Y est un groupe ou un bras d'espacement qui est constitué par un radical organique bivalent, choisi parmi les groupes alkylène linéaires ou ramifiés en $C_1$ à $C_{20}$, contenant éventuellement une ou plusieurs doubles liaisons et/ou étant éventuellement interrompus par un ou plusieurs hétéroatomes, tels que l'oxygène, l'azote, le soufre ou le phosphore, parmi les groupes cycloalkylène en $C_5$-$C_8$ ou parmi les groupes arylène en $C_6$ à $C_{14}$, lesdits groupes alkylène, cycloalkylène ou arylène étant éventuellement substitués par des groupes alkyle, aryle ou sulfonate;
- Z est un groupe fonctionnel susceptible de se lier de façon covalente avec une substance biologique ;
- R est un groupe méthyle ou représente le groupe -Y-Z ;
- R′ est l'hydrogène ou un groupe -COOR″ dans lequel R″ est un groupe alkyle en $C_1$ à $C_{10}$ et représente de préférence le groupe méthyle, éthyle ou tertiobutyle ou bien R′ est un groupe -CO-NH-Y-Z.

**14.** Procédé selon l'une quelconque des revendications 1 à 8, caractérisé en ce que le composé fluorescent donneur est un cryptate d'europium et en ce que le composé fluorescent accepteur est choisi parmi l'allophycocyanine, l'allophycocyanine B, la C phycocyanine ou la R phycocyanine.

**15.** Procédé selon l'une quelconque des revendications 1 à 8, caractérisé en ce que le composé fluorescent donneur est un cryptate de terbium et en ce que le composé fluorescent accepteur est choisi parmi les rhodamines, la thionine, la R phycocyanine, la phycoerythrocyanine, la C phycoerythrine, la B phycoerythrine ou la R phycoerythrine.

**Patentansprüche**

1. Verfahren zur Verstärkung des Emissionssignals einer lumineszierenden Verbindung, die als Donorverbindung bei der quantitativen Bestimmung einer zu analysierenden Substanz durch Lumineszenzmessung verwendet wird,
bei dem ferner eine lumineszierende Akzeptorverbindung eingesetzt wird und das emittierte Signal der Akzeptorverbindung nach Anregung der Donorverbindung bei ihrer Anregungswellenlänge gemessen wird,
dadurch gekennzeichnet, daß
die Donorverbindung eine geringe Gesamt-Quantenausbeute aufweist und die Quantenausbeute der Strahlungsdesaktivierung vom Emissionsniveau der Donorverbindung kleiner ist als die Quantenausbeute der Akzeptorverbindung.

2. Verfahren zur Verstärkung des Emissionssignals eines Seltenerdmetallkryptats oder Seltenerdmetallchelats, das als Donorverbindung bei der quantitativen Bestimmung einer zu analysierenden Substanz durch Lumineszenzmessung verwendet wird,
bei dem ferner eine lumineszierende Akzeptorverbindung eingesetzt wird und das emittierte Signal der Akzeptorverbindung nach Anregung des Seltenerdmetallkryptats oder Seltenerdmetallchelats bei seiner Anregungswellenlänge gemessen wird,
dadurch gekennzeichnet, daß
das Seltenerdmetallkryptat oder Seltenerdmetallchelat eine geringe Gesamt-Quantenausbeute aufweist und die Quantenausbeute der Strahlungsdesaktivierung vom Emissionsniveau des Seltenerdmetalls kleiner ist als die Quantenausbeute der Akzeptorverbindung.

3. Homogenes Verfahren zur qualitativen und/oder quantitativen Bestimmung einer zu analysierenden Substanz in einem Medium, in dem sie enthalten sein kann, durch Lumineszenzmessung unter Einsatz des Produkts der Reaktion zwischen der zu analysierenden Substanz und mindestens einem entsprechenden Rezeptor, das folgende Schritte umfaßt:
1) Zugabe eines ersten Reaktanten, der aus mindestens einem Rezeptor der zu analysierenden Substanz besteht, zum Medium,
2) Zugabe eines zweiten, unter der zu analysierenden Substanz und mindestens einem ihrer Rezeptoren ausgewählten Reaktanten, wobei einer der beiden Reaktanten an eine lumineszierende Donorverbindung, die aus einem Seltenerdmetallkryptat oder Seltenerdmetallchelat besteht, und der andere Reaktant an eine lumineszierende Akzeptorverbindung gekuppelt sind,
wobei die Reihenfolge der Zugabe der Reaktanten umgekehrt werden kann,
und, nach Anregung des Gemisches mit einer Lichtquelle bei der Anregungswellenlänge der lumineszierenden Donorverbindung,
3) Messung des von der lumineszierenden Akzeptorverbindung emittierten Signals,
dadurch gekennzeichnet, daß das als Donorverbindung eingesetzte Seltenerdmetallkryptat oder Seltenerdmetallchelat eine geringe Gesamt-Quantenausbeute aufweist und die Quantenausbeute der Strahlungsdesaktivierung vom Emissionsniveua des Seltenerdmetalls kleiner ist als die Quantenausbeute der Akzeptorverbindung.

4. Verfahren nach Anspruch 3, das ein Überschußverfahren darstellt,
gekennzeichnet durch folgende Schritte:
1) Zugabe eines ersten Reaktanten, der aus mindestens einem Rezeptor der zu analysierenden Substanz besteht, der an eine lumineszierende Donorverbindung gekuppelt ist, die aus einem Seltenerdmetallkryptat oder Seltenerdmetallchelat besteht, zu dem Medium, das die zu analysierende Substanz enthält,
2) Zugabe eines zweiten, aus einem oder mehreren anderen Rezeptoren der zu analysierenden Substanz bestehenden Reaktanten, wobei der zweite Reaktant an eine lumineszierende Akzeptorverbindung gekuppelt ist,
3) Inkubation des Mediums nach jeder Zugabe von Reaktanten oder nach Zugabe beider Reaktanten,
4) Anregung des resultierenden Mediums bei der Anregungswellenlänge der lumineszierenden Donorverbindung
und
5) Messung des von der lumineszierenden Akzeptorverbindung emittierten Signals.

5. Verfahren nach Anspruch 3, das ein kompetitives Verfahren darstellt, gekennzeichnet durch folgende Schritte:
   1) Zugabe eines ersten Reaktanten, der aus einem Rezeptor der zu analysierenden Substanz besteht, der an eine lumineszierende Donorverbindung gekuppelt ist, die aus einem Seltenerdmetallkryptat oder Seltenerdmetallchelat besteht, zu dem Medium, das die zu analysierende Substanz enthält,
   2) Zugabe eines zweiten Reaktanten, der aus der an eine lumineszierende Akzeptorverbindung gekuppelten zu analysierenden Substanz besteht,
   3) Inkubation des Mediums nach jeder Zugabe von Reaktanten oder nach Zugabe beider Reaktanten,
   4) Anregung des resultierenden Mediums bei der Anregungswellenlänge der lumineszierenden Donorverbindung
   und
   5) Messung des von der lumineszierenden Akzeptorverbindung emittierten Signals.

6. Verfahren nach Anspruch 3, das ein kompetitives Verfahren darstellt, gekennzeichnet durch folgende Schritte:
   1) Zugabe eines ersten Reaktanten, der aus einem Rezeptor der zu analysierenden Substanz besteht, der an eine lumineszierende Akzeptorverbindung gekuppelt ist, zu dem Medium, das die zu analysierende Substanz enthält, die bestimmt werden soll,
   2) Zugabe eines zweiten Reaktanten, der aus der zu analysierenden Substanz besteht, die an eine lumineszierende Donorverbindung gekuppelt ist, die aus einem Seltenerdmetallkryptat oder Seltenerdmetallchelat besteht,
   3) Inkubation des Mediums nach Zugabe jedes Reaktanten oder nach Zugabe beider Reaktanten,
   4) Anregung des resultierenden Mediums bei der Anregungswellenlänge der lumineszierenden Donorverbindung
   und
   5) Messung des von der lumineszierenden Akzeptorverbindung emittierten Signals.

7. Verfahren nach einem der Ansprüche 3 bis 6, dadurch gekennzeichnet, daß der erste und der zweite Reaktant dem die zu analysierende Substanz enthaltenden Medium gleichzeitig zugegeben werden.

8. Verfahren nach Anspruch 3 oder 4, dadurch gekennzeichnet, daß ein einziger Rezeptor der zu analysierenden Substanz verwendet wird, der entweder an die lumineszierende Donorverbindung oder an die lumineszierende Akzeptorverbindung gekuppelt ist.

9. Verfahren nach einem der Ansprüche 1 bis 8, dadurch gekennzeichnet, daß die lumineszierende Donorverbindung ein Chelat oder ein Kryptat von Terbium oder Europium ist.

10. Verfahren nach Anspruch 9, dadurch gekennzeichnet, daß die Donorverbindung ein Seltenerdmetallkryptat ist, das aus mindestens einem Seltenerdmetallsalz besteht, das mit einer makropolycyclischen Verbindung der allgemeinen Formel komplexiert ist,

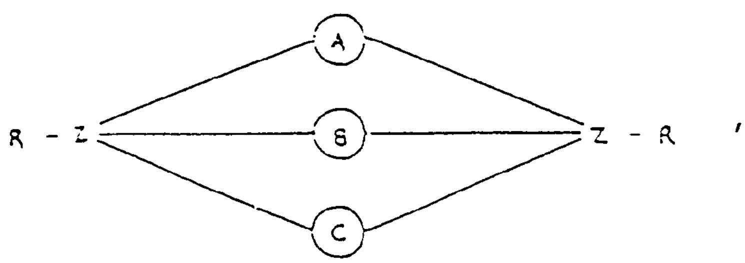

in der bedeuten:

| | |
|---|---|
| Z | ein drei- oder vierwertiges Atom, wie Stickstoff, Kohlenstoff oder Phosphor, |
| R | keinen Substituenten oder Wasserstoff, Hydroxy, Amino oder eine Kohlenwasserstoffgruppe und |
| A, B und C | unabhängig voneinander Kohlenwasserstoffketten, die ggfs. ein oder mehrere Heteroatome enthalten und ggfs. durch einen Heteromakrocyclus unterbrochen sind, wobei mindestens eine der Gruppen A, B und C ferner mindestens eine Molekülgruppe |

trägt oder im wesentlichen aus ihr besteht, die eine Triplett-Energie besitzt, die größer ist als die Energie des Emissionsniveaus des komplexgebundenen Seltenerdmetallions.

**11.** Verfahren nach Anspruch 9, dadurch gekennzeichnet, daß das Seltenerdmetallkryptat mit einer makropolycyclischen Verbindung der allgemeinen Formel komplexiert ist,

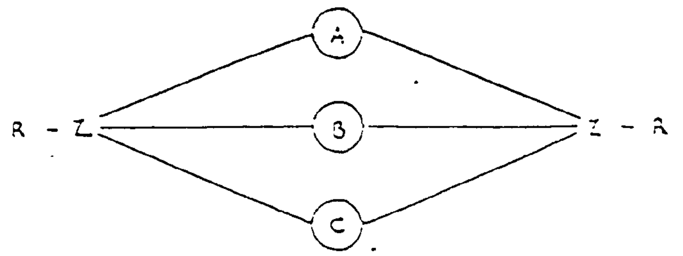 ,

in der bedeuten:
A, B und C:

$- C_2H_4 - X_1 - C_6H_4 - X_2 - C_2H_4 -$

$- C_2H_4 - X_1 - CH_2 - C_6H_4 - CH_2 - X_2 - C_2H_4 -$,

wobei $X_1$ und $X_2$, die gleich oder voneinander verschieden sind, Sauerstoff, Stickstoff oder Schwefel bezeichnen,

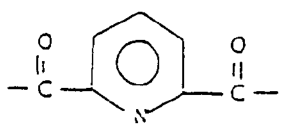 : 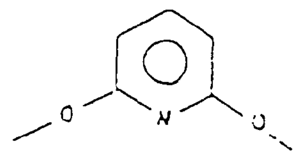

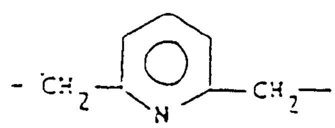

oder

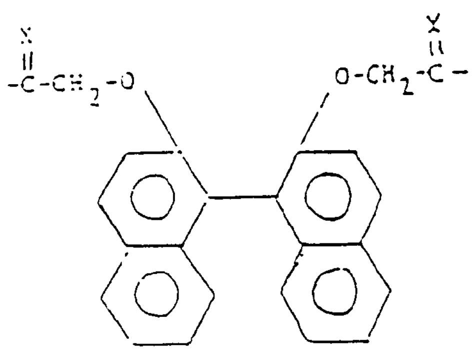

worin X Sauerstoff oder Wasserstoff bedeutet.

**12.** Verfahren nach Anspruch 9 oder 10, dadurch gekennzeichnet, daß die Donorverbindung das Terbiumkryptat Tb-Tris(bipyridin) oder das Europiumkryptat Eu-Tris(bipyridin) ist.

**13.** Verfahren nach Anspruch 8, dadurch gekennzeichnet, daß die lumineszierende Donorverbindung ein

Seltenerdmetallkryptat ist, das aus mindestens einem Seltenerdmetallsalz besteht, das mit einer makropolycyclischen Verbindung der Formel I oder II komplexiert ist,

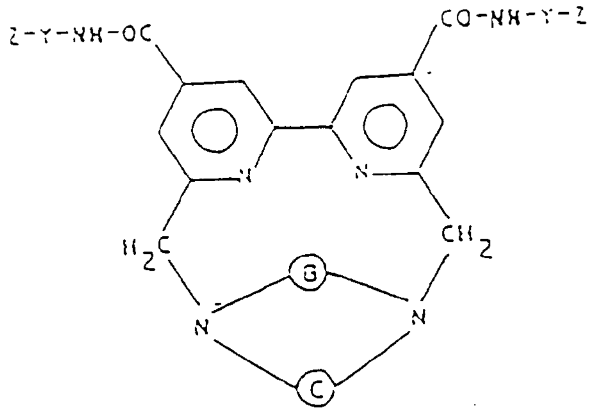

(I),

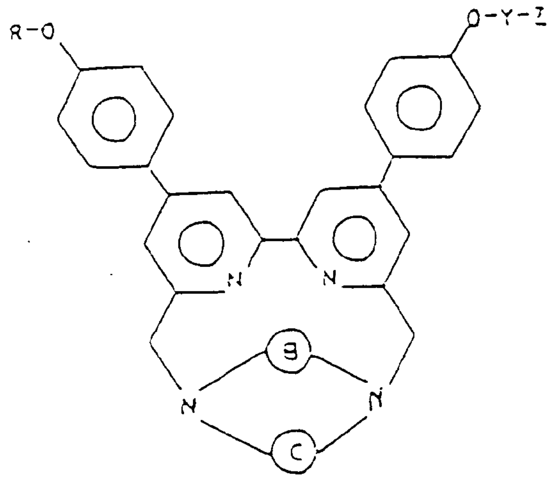

(II),

worin bedeuten:

- das Ringsystem der Formel

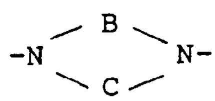

eine der folgenden cyclischen Gruppen:

1)

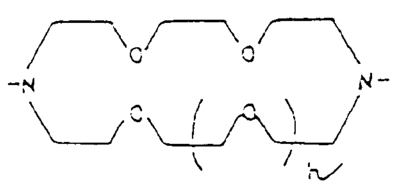

n = 0 oder 1
Makrocyclus $[N_2O_4]$
bzw. Cyclus (22)
Makrocyclus $[N_2O_3]$
bzw. Cyclus (21)

2) 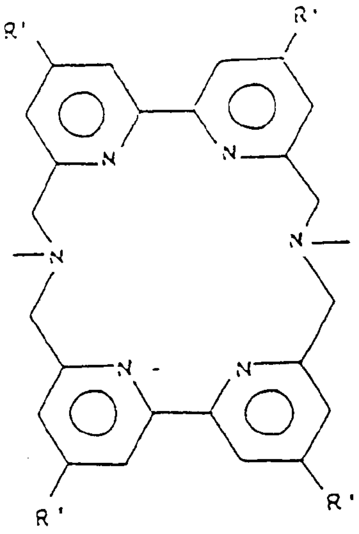 makrocyclisches Bis(bipyridin),

- Y eine Gruppe oder eine Spacergruppe, die aus einer zweiwertigen organischen Gruppe besteht, die ausgewählt ist unter geradkettigen und verzweigten $C_{1-20}$-Alkylgruppen, die ggfs. eine oder mehrere Doppelbindungen aufweisen und/oder ggfs. durch ein oder mehrere Heteroatome, wie Sauerstoff, Stickstoff, Schwefel und Phosphor, unterbrochen sind, $C_{5-8}$-Cycloalkylengruppen und $C_{6-14}$-Arylengruppen, wobei die Alkylengruppen, Cycloalkylengruppen und Arylengruppen ggfs. mit Alkylgruppen, Arylgruppen oder Sulfonatgruppen substituiert sind,
- Z eine funktionelle Gruppe, die befähigt ist, eine kovalente Bindung mit einer biologischen Substanz einzugehen,
- R Methyl oder die Gruppierung -Y-Z und
- R′ Wasserstoff oder eine Gruppe -COOR″, in der R″ $C_{1-10}$-Alkyl und vorzugsweise Methyl, Ethyl oder t-Butyl darstellt, oder die Gruppe -CO-NH-Y-Z.

**14.** Verfahren nach einem der Ansprüche 1 bis 8, dadurch gekennzeichnet, daß die fluoreszierende Donorverbindung ein Europiumkryptat ist und die fluoreszierende Akzeptorverbindung unter Allophycocyanin, Allophycocyanin B, C-Phycocyanin und R-Phycocyanin ausgewählt ist.

**15.** Verfahren nach einem der Ansprüche 1 bis 8, dadurch gekennzeichnet, daß die fluoreszierende Donorverbindung ein Terbiumkryptat ist und die fluoreszierende Akzeptorverbindung unter den Rhodaminen, Thionin, R-Phycocyanin, phycoerythrocyanin, C-Phycoerythrin, B-phycoerythrin und R-Phycoerythrin ausgewählt ist.

## Claims

**1.** Method of amplifying the emission signal of a luminescent compound used as a donor compound in a luminescent assay of an analyte, in which a luminescent acceptor compound is also used, and in which the emission signal of the acceptor compound is measured after excitation of the donor compound at its excitation wavelength, characterized in that the donor compound possesses a low overall quantum yield and in that the quantum yield of radiative deactivation of the emission level of the donor is lower than the quantum yield of the acceptor.

**2.** Method of amplifying the emission signal of a rare earth chelate or cryptate used as a donor compound in a luminescent assay of an analyte, in which a luminescent acceptor compound is also used, and in which the emission signal of the acceptor compound is measured after excitation of the rare earth chelate or cryptate at its excitation wavelength, characterized in that the rare earth chelate or cryptate possesses a low overall quantum yield and in that the quantum yield of radiative deactivation of the emission level of the rare earth is lower than the quantum yield of the acceptor.

3. Homogeneous luminescent method of detecting and/or determining an analyte in a medium in which it may be present, by revealing the product of the reaction of the analyte with at least one corresponding receptor, said method consisting in:
   1) adding to said medium a first reagent consisting of at least one receptor for said analyte,
   2) adding a second reagent selected from the analyte or at least one of its receptors, one of the two reagents being coupled with a luminescent donor compound consisting of a rare earth chelate or cryptate and the other reagent being coupled with a luminescent acceptor compound, it being possible for the order of addition of the reagents to be reversed, and, after excitation of the mixture with a light source at the excitation wavelength of the luminescent donor compound,
   3) measuring the emission signal of the luminescent acceptor compound,

   characterized in that the rare earth chelate or cryptate used as the donor compound possesses a low overall quantum yield and in that the yield of radiative deactivation of the emission level of the rare earth is lower than the quantum yield of the acceptor.

4. Method according to claim 3 which consists in an excess method, characterized in that it consists in:
   1) adding, to said medium containing the analyte being tested, a first reagent consisting of at least one receptor for said analyte, coupled with a luminescent donor compound consisting of a rare earth chelate or cryptate,
   2) adding a second reagent consisting of one or more different receptors for said analyte, said second reagent being coupled with a luminescent acceptor compound,
   3) incubating said medium after each addition of reagents or after the addition of the two reagents,
   4) exciting the resulting medium at the excitation wavelength of the luminescent donor compound,
   5) measuring the signal emitted by the luminescent acceptor compound.

5. Method according to claim 3 which consists in a competitive method, characterized in that it consists in:
   1) adding, to said medium containing the analyte being tested, a first reagent consisting of a receptor for said analyte, coupled with a luminescent donor compound consisting of a rare earth chelate or cryptate,
   2) adding a second reagent consisting of the analyte coupled with a luminescent acceptor compound,
   3) incubating said medium after each addition of reagents or after the addition of the two reagents,
   4) exciting the resulting medium at the excitation wavelength of the luminescent donor compound,
   5) measuring the signal emitted by the luminescent acceptor compound.

6. Method according to claim 3 which consists in a competitive method, characterized in that it consists in:
   1) adding, to said medium containing the analyte being tested, a first reagent consisting of a receptor for said analyte, said receptor being coupled with a luminescent acceptor compound,
   2) adding a second reagent consisting of the analyte coupled with a luminescent donor compound consisting of a rare earth chelate or cryptate,
   3) incubating said medium either after the addition of each reagent or after the addition of the two reagents,
   4) exciting the resulting medium at the excitation wavelength of the luminescent donor compound,
   5) measuring the signal emitted by the luminescent acceptor compound.

7. Method according to any one of claims 3 to 6, characterized in that the first and second reagents are added simultaneously to the medium containing the analyte being tested.

8. Method according to any one of claims 3 or 4, characterized in that a single receptor for the analyte, which is coupled either with the luminescent donor compound or with the luminescent acceptor compound, is used.

9. Method according to any one of claims 1 to 8, characterized in that the luminescent donor compound is a chelate or cryptate of terbium or europium.

10. Method according to claim 9, characterized in that the donor compound is a rare earth cryptate consisting of at least one rare earth salt complexed by a macropolycyclic compound of the general formula

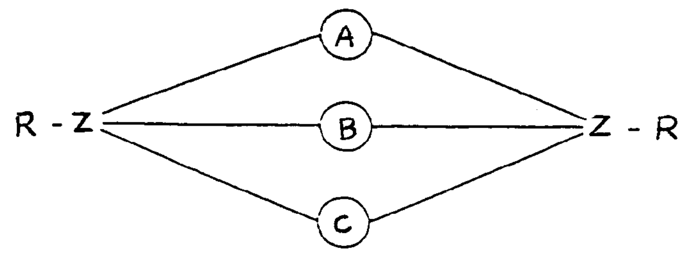

in which Z is a trivalent or tetravalent atom such as nitrogen, carbon or phosphorus, R is nothing, hydrogen, the hydroxyl group, an amino group or a hydrocarbon radical and the divalent radicals Ⓐ, Ⓑ and Ⓒ independently of one another are hydrocarbon chains which may or may not contain one or more heteroatoms and may or may not be interrupted by a heteromacrocycle, at least one of the radical Ⓐ, Ⓑ or Ⓒ additionally containg at least one molecular unit or essentially consisting of a molecular unit, said molecular unit possessing a greater triplet energy than the emission level of the complexed rare earth ion.

**11.** Method according to claim 9, characterized in that the rare earth cryptate compound is complexed by a macropolycyclic compound of the general formula

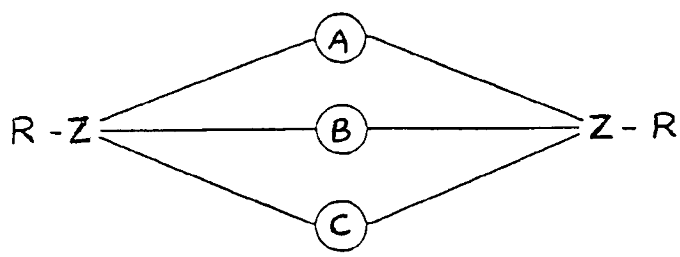

in which Ⓐ, Ⓑ and Ⓒ are defined as follows:

$-C_2H_4- X_1 - C_6H_4 - X_2 - C_2H_4 -$

$-C_2H_4- X_1 - CH_2 - C_6H_4 - CH_2 - X_2 - C_2H_4 -$

$X_1$ and $X_2$, which can be identical or different, denote oxygen, nitrogen or sulfur;

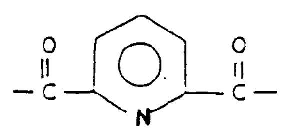 ; 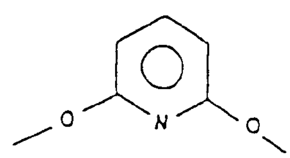

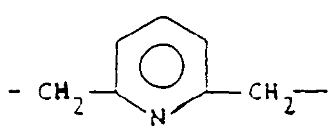

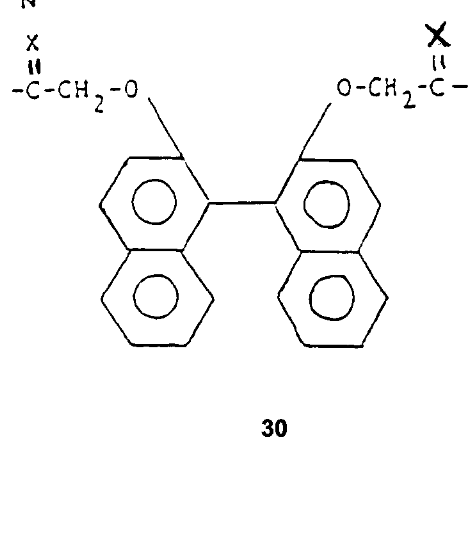

X being oxygen or hydrogen.

**12.** Method according to claims 9 or 10, characterized in that the donor compound is the terbium cryptate Tb trisbipyridine or the europium cryptate Eu trisbipyridine.

**13.** Method according to claim 8, characterized in that the luminescent donor compound is a rare earth cryptate consisting of at least one rare earth salt complexed by a macropolycyclic compound of formula I or II:

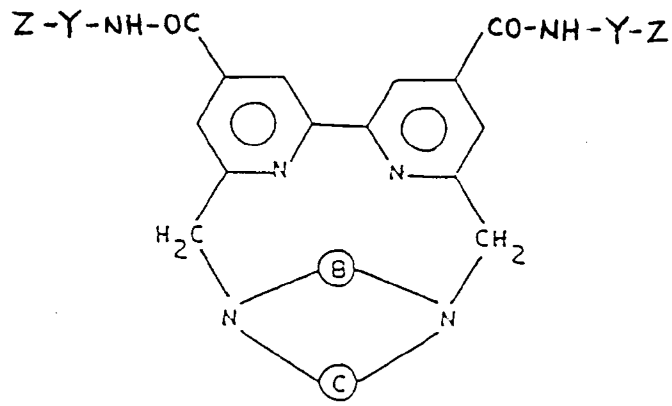

I

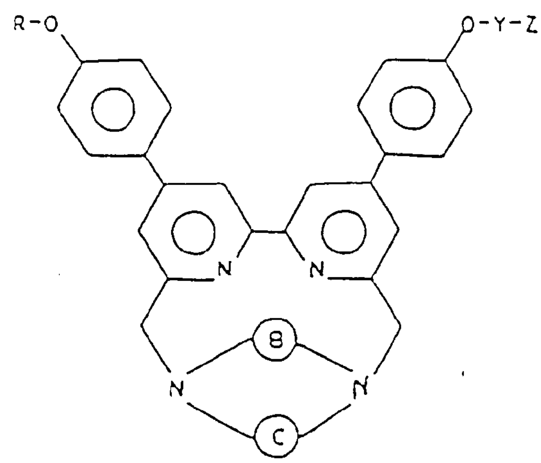

II

in which

- the ring of the formula

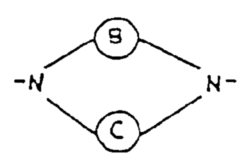

is one of the following rings:

1) 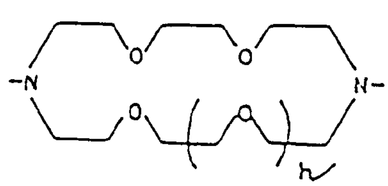

n = 0 or 1

$[N_2O_4]$ macrocycle or (22) ring

$[N_2O_3]$ macrocycle or (21) ring

2) 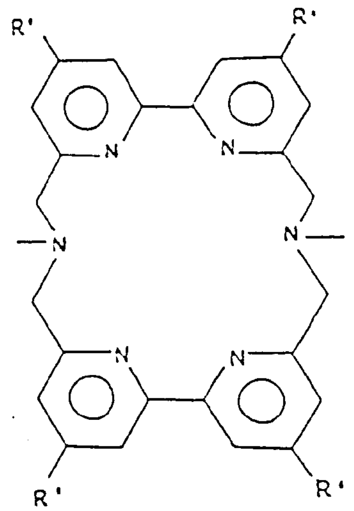 bisbipyridine macrocycle

- Y is a group or spacer arm which consists of a divalent organic radical selected from linear or branched $C_1$ to $C_{20}$ alkylene groups which may or may not contain one or more double bonds and/or may or may not be interrupted by one or more heteroatoms such as oxygen, nitrogen, sulfur or phosphorus, from $C_5$-$C_8$ cycloalkylene groups or from $C_6$ ot $C_{14}$ arylene groups, said alkylene, cycloalkylene or arylene groups being unsubstituted or substituted by alkyl, aryl or sulfonate groups;
- Z is a functional group capable of bonding covalently with a biological substance;
- R is a methyl group or the group -Y-Z;
- R′ is a hydrogen or a group -COOR″, in which R″ is a $C_1$ to $C_{10}$ alkyl group and preferably represents the methyl, ethyl or tert-butyl group, or alternatively R′ is a group -CO-NH-Y-Z.

**14.** Method according to any one of claims 1 to 8, characterized in that the fluorescent donor compound is an europium cryptate and in that the fluorescent acceptor compound is selected from allophycocyanine, allophycocyanine B, C phycocyanine or R phycocyanine.

**15.** Method according to any one of claims 1 to 8, characterized in that the fluorescent donor compound is a terbium cryptate and in that the fluorescent acceptor compound is selected from rhodamines, thionine, R phycocyanine, phycoerythrocyanine, C phycoerythrin, B phycoerythrin or R phycoerythrin.